# EUROPEAN PATENT SPECIFICATION

(11) **EP 2 844 202 B1**
(45) Date of publication and mention of the grant of the patent: **20.07.2016**
(21) Application number: 12721787.5
(22) Date of filing: 01.05.2012
(51) Int. Cl.: A61F 13/00

(54) **A WOUND DRESSING LAMINATE COMPRISING A LAYER IMPREGNATED WITH AN ANTIMICROBIAL AGENT, A METHOD OF MANUFACTURING THE WOUND DRESSING LAMINATE AND WOUND DRESSINGS MADE OF THE WOUND DRESSING LAMINATE**
WUNDVERBANDLAMINAT MIT EINER MIT EINEM ANTIMIKROBIELLEN WIRKSTOFF IMPRÄGNIERTEN SCHICHT, VERFAHREN ZUR HERSTELLUNG DES WUNDVERBANDLAMINATS UND AUS DEM WUNDVERBANDLAMINAT HERGESTELLTE WUNDVERBÄNDE
STRATIFIÉ DE PANSEMENT POUR PLAIE COMPRENANT UNE COUCHE IMPRÉGNÉE D'UN AGENT ANTIMICROBIEN, PROCÉDÉ DE FABRICATION DU STRATIFIÉ DE PANSEMENT POUR PLAIE ET PANSEMENTS POUR PLAIE FAITS DU STRATIFIÉ DE PANSEMENT POUR PLAIE

(43) Date of publication of application: 11.03.2015
(73) Proprietor: Pharmaplast SAE, Alexandria 23512 (EG)
(72) Inventor: FARES, Wassim, Alexandria 23512 (EG); AZIZ, Dina, Alexandria 23512 (EG)
(74) Representative: Holme Patent A/S
(86) International application number: PCT/EP2012/057956
(87) International publication number: WO 2013/164016

(56) References cited:
- WO-A1-2007/085884
- WO-A2-03/033041
- WO-A2-2008/005532
- GB-A- 2 462 678
- US-A1- 2011 208 101

## Description

The present invention relates to a wound dressing laminate according to claim 1 comprising a layer impregnated with an antimicrobial agent, a method of manufacturing according to claim 8 the wound dressing laminate and wound dressings made of the wound dressing laminate.

Many wounds are very susceptible to infections, and various kinds of wound dressings are offered to minimize such risk during healing.

Some wounds produce exudates, in which case the healing process can be slowed down. In case of wound infection and/or oedema, exudate production and leakage from the wound increases. Proteases in the exudates digests new tissue making treatment time-consuming and is responsible for painful maceration of the surrounding healthy skin. Some exudating wounds may be so difficult to treat that they become chronic.

A known means to accelerate healing is sucking exudate away from the wound by means of an absorbent dressing. Effective exudates management that prevent maceration and infection is however a difficult task. Exudates should be absorbed and retained by the absorbent dressing while still keeping the wound moist to promote healing, which dressing must not become a growth medium for infectious matter. To reduce risk of infections some wound dressings include an antimicrobial component such as silver or povidone-iodine that passes into the wound when interacting with exudates. The antimicrobial component may be soaked in a foam dressing or be applied to gauze or knitted fabric. A huge disadvantage of known medicated wound dressing are however that they eventually stick to the wound when exudates deplete. Then it becomes very painful to remove the medicated would dressings from the dry wound, and the vulnerable new tissue often disintegrates.

Examples of known low adherent wound dressings for exudating wounds include for example Mepitel® and Inadine®.

Mepitel® is a porous low-adherent wound dressing manufactured by Mölnlycke Health Care. The wound dressing has a wound contact layer of a flexible non-absorbent silicone-coated polyamide net. The silicone coating serves to keep Mepitel® attached to the peri-wound area. However Mepitel® lacks means to prevent infection.

An alternative wound dressing configured to prevent bacterial, protozoal and fungal infections is Inadine® manufactured by Johnson & Johnson Medical Ltd. Inadine® is a colored, low-adherent knitted viscose fabric impregnated with 10% povidone iodine in a water-soluble polyethylene glycol. Fading of color from orange-brown to white indicates gradually loss of antiseptic properties until complete povidone iodine depletion. The medicated knitted fabric is a mesh having good release properties of antimicrobial povidone iodine and it is generally low-adherent to wound but adheres to the wound as soon as exudates deplete. Inadine® needs to be covered with a separate, secondary, absorbent sponge dressing that retains exudates. This layered structure is secured to the skin by means of tape and is changed often to avoid that cytotoxic povidone-iodine from the fabric is in direct contact with the wound and accumulate in the wound bed. Depletion of exudates cannot be monitored to ensure timely replacement with same or other dressing, and to prevent sticking and avoiding potential ingrowth of new tissue in knitted impregnated fabric.

US patent no. 5,147,338 relates to a medicated, low-adherent laminate dressing comprising a conformable elastomeric apertured wound-facing film layer, an intermediate conformable medicated absorbent hydrophilic foam layer acting like a sponge, and an outer layer being a continuous moisture vapour transmitting conformable film. The wound-facing film layer needs to be elastically extensible to adjust to dimensional changes when the absorbent foam layer expands on exudate uptake. The medicament of the foam layer can e.g. be povidone-iodine, which is incorporated prior to foaming the components of the absorbent layer or is applied by soaking the foam layer in a medicament solution. Soaking of foam layer makes it expand so that the absorption capacity is reduced. This known dressing does not allow superabsorbent fibers and/or superabsorbent powders to be incorporated in the absorbent foam layer because their absorbent property cannot be restored to satisfactory after soaking in a medicament solution or survive foaming. Another disadvantage is that medicament may leak considerable from the medicated foam as soon as the foam layer begins to absorb exudate or liquid matter in general.

From US patent no. US 6,471,982 is known an absorbent sheet dressing comprising a mixture of textile fibres, gel-forming fibres and antimicrobial agent. All components are combined in same layer by carding or air-laying. This known wound dressing addresses the problem that when new tissue is formed as part of the healing process, it engulfs the fibres of a fiber-containing wound dressing, and is torn when the dressing is removed, thereby causing wound injury. Non-adherency of this known wound dressing relies solely in that when the wound dressing comes in contact with the wound the gel-forming fibres will absorb exudate causing these gel-forming fibres to swell and form a gel. The gel renders the wound dressing sufficiently slippery to prevent adherence of the dressing to the newly formed tissue. This known dressing puts antimicrobial agent in direct contact with the wound, which is not always acceptable due to cytotoxity. Moreover, eventually the wound becomes dry and there remains a considerable risk that new tissue grows into the fiber sheet during especially late stages of healing. US patent application no. 2011/0171284 describes the treatment of wounds by use of a medical dressing comprising povidone-iodine and sucrose. Povidone iodine solution and sucrose are gelled using a gelling agent, such as gums, polysaccharides, hydrocolloids and alginates to create a composition with increased viscosity. The composition keeps the sucrose positioned in the gelled povidone iodine solution in a biologically active manner. The gel is impregnated on a fibrous wound dressing or can be used directly at the wound for standardized dosaging. This dressing has only minor absorbent capacity left. A further disadvantage is that the gel gradually disintegrated and gel residues accumulate in the wound bed to prolong healing.

Others wound dressings are for example described in US20110208101, WO2008005532, GB2462678, and WO03033401 The above disadvantages and short-comings of prior art dressing have inspired the inventors of the present invention to new solutions.

In a first aspect is provided a wound dressing laminate and wound dressings of the kind mentioned in the opening paragraph to be used in the management of wounds, in particular exudating wounds, to prevent or inhibit infection and growth of microorganisms in a wound.

In a second aspect of the present invention is provided a wound dressing laminate and wound dressings of the kind mentioned in the opening paragraph, which does not grow into the wound, does not stick or adhere to an exudating wound even if depleted of exudate, or just sticks or adheres to such a depleted wound to a neglible extent allowing removal of the dressing without pain and disintegration of new tissue.

In a third aspect of the present invention is provided a wound dressing laminate and wound dressings of the kind mentioned in the opening paragraph that eliminate, or at least reduces, direct contact between medicament and wound, and lead exudates away from the wound.

In a fourth aspect of the present invention is provided a wound dressing laminate and wound dressings of the kind mentioned in the opening paragraph as alternatives to known wound dressing laminates and wound dressings.

In a fifth aspect of the present invention is provided a wound dressing laminate and wound dressings that do not push exudate or infectious matter back to the wound bed.

In a sixth aspect of the present invention is provided a wound dressing laminate and wound dressings that do not allow fluid to wick across the surface of the dressings so that fluid affects healthy tissue and causes maceration.

The novel and unique whereby these and other aspect are achieved according to the present invention consist in that said impregnated layer is inserted between an apertured wound-contacting layer and an absorbent non-woven layer.

Within the scope of the present invention a "non-woven layer" should be understood as a layer of fibers made coherent by a process not involving weaving or knitting.

The term "laminate" is used for layered structures made by uniting two or more layers of materials firmly together, e.g. by gluing or mechanical treatment.

The term "apertured" means any passageway, channel og through-going opening through that allow passage of an antimicrobial agent into the wound.

In the present context the term "non-adherent" means "not to adhere, stick or hold on to a subjacent surface", in particular a surface such as a wound or wound bed, more in particular not sticking to the dried secretions of a wound or to the wound bed itself or being engulfed by new tissue, at least more than to an inferior and painless extent upon removal of the dressing made of the wound dressing laminate.

The term "antimicrobial agent" is used for a chemical substance that discourages and/or inhibits the growth and reproduction of microorganisms. Antibiotics and disinfectants are excluded as antimicrobial agents within the scope of the present invention.

Adherence of wound bandages and wound dressings to underlying tissue constitutes a particular clinical problem, in particular when exudate depletes and the wound becomes dry. This problem becomes of increased concern when the wound dressings are of the kind that includes medicaments, e.g medicaments to facilitate prevention or inhibiting of infection and growth of microorganisms, which medicaments often are cytotoxic. High concentrations of medicament in direct wound contact are undesired. According to the present invention an apertured wound-contacting layer and an absorbent non-woven layer are united by lamination to opposite sides of a layer impregnated with an antimicrobial agent.

By selecting an appropriate substantially or entirely non-adherent apertured wound-contacting layer to unite with the layer impregnated with antimicrobial agent, adherence to the wound can be avoided and the layer impregnated with antimicrobial agent can be kept spaced from the wound while the antimicrobial agent still is released to act as an antiseptic. The antimicrobial agent is dissolved in the presence of exudates.

The absorbent non-woven layer absorbs exudates in controlled manner allowing the wound to be kept moist. The antimicrobial agent containing wound dressing laminate according to the present invention induces very fast healing, need not be changed as often as conventional wound dressings, and is easy to remove in a painless manner from the healed or partly healed wound.

The antimicrobial agent is povidone-iodine. Elemental iodine is liberated from povidone-iodine, and iodine then leaches out from the dressing via the apertured wound-contacting layer to reach the wound bed to do its antimicrobial action.

It should be emphasized that antimicrobial agents may be heat-sensitive, and that any lamination step that either uses or produces heat will impair antiseptic properties. In particular povidone-iodine is heat-sensitive, so technical challenges and complications relating to manufacturing laminate dressings with layers containing povidone-iodine, without destroying the sought-after desirable properties of the medicament, have hitherto not encouraged to introduce such povidone-iodine impregnated layers, into laminate dressings. In particular have such impregnated layer not been included in laminate dressings of reduced susceptibility of adherence to wound and reduced direct exposure of cytotoxic antimicrobial medicament to wound.

The laminate's impregnated center layer serves as a medicament reservoir layer with selected concentration of antimicrobial agent to be liberated into the wound upon dissolution by the action of exudates or other fluids. Contrary to when using known medicament impregnated layers, which normally is susceptible to grow into the wound and to stick to the wound, the wound dressing laminate according to the present invention is beneficially kept out of direct contact with the wound by means of the apertured wound-contacting layer, which apertured wound-contacting layer also keeps the antimicrobial agent under sustained release until depletion. The apertured wound-contacting layer is a kind of blockade layer between wound and impregnated layer but allows the antimicrobial agent to be gradually released and delivered to the wound in a controlled manner.

Suitable medical grade apertured wound-contacting layers are made of polyolefins that can be manufactured into very thin, lightweight, soft and flexible film layers. Polyolefin films have very good chemical stability and are resistant to most acids, bases, and salt solutions. Accordingly, exudates cannot dissolve the polyolefin film itself. Polyolefin film layers may be waxy to touch and have a surface on which adhesion is very difficult. They do not stick to a wound, or only stick to a negligible extent. A preferred polyolefin is polyethylene or polypropylene that possesses the above-described beneficial properties.

An alternative to a polyolefin film may be a polyester film or a polyurethane film. Polyurethane films are also chemically resistant to most acids, bases, and salt solutions. A polyurethane film even has elastomeric properties and can be stretch slightly if the absorbent layer and the impregnated layers allow so without deteriorating the coherency of the lamination. Medical grade breathable polyurethane films may be incorporated in the wound dressing laminate according to the present invention if considered expedient.

No skin-contacting adhesive or skin-contacting glue is applied to, coated on or otherwise used on the skin-contacting surface of the apertured wound-contacting layer.

In a preferred embodiment the apertured wound-contacting layer is an apertured film structure in form of a perforated film, a net, a scrim or a mesh to allow the antimicrobial agent to pass from the impregnated layer into the wound.

In summary the wound dressing laminate has a substantially non-adherent apertured wound-containing layer intended for facing the wound bed to prevent adherence to the wound, and traumatic or painful removal of a corresponding dressing made of said laminate. The apertured wound-contacting layer also regulates the absorption of exudates which in turn affects the release of antimicrobial agent, such as release of elemental iodine from the povidone-iodine complex. This prevents or at least reduces cytotoxic exposure and skin irritation that might be caused by excess antimicrobial agent, in particular in case of iodine. In case of povidone-iodine the apertured wound-contacting layer prevents fast depletion of iodine from the povidone-iodine complex which in turn prolongs the wear time of a dressing.

The wound dressing laminate according to the present invention includes an absorbent non-woven layer that comprises one or more of the absorbent components selected from the group comprising viscose fibers, polyolefin fibers, superabsorbent fibers and gel-forming fibers or combinations thereof.

Non-wovens are inexpensive and have superior absorbent capacity, which can be further improved by adding a further absorbent component, such as absorbent fibers. The superabsorbent fibers and gel-forming fibers can retain substantial amounts of fluid, so that the wound bed maintains wet but is not soaked in exudates. The absorbent capacity of the fibers contributes in controlling the volume of exudates accumulating in the wound bed and therefore also the concentration of antimicrobial agent present in the wound bed. The selected content and composition of highly absorbent fibers may also prevent leakage of antimicrobial agent dissolved in exudates from flowing away from the wound bed before the antimicrobial agent has acted beneficially.

Viscose (rayon) fibers are made of regenerated wood cellulose and are the most absorbent of all cellulose fibers. Viscose fibers are inexpensive, can be made into very soft webs, breathe like cotton fibers, but has higher absorbent capacity, and are easier to work with.

Viscose fibers are however fairly weak and a better web is obtained if the viscose fibers are blended with other fibers, such as polyolefin fibers, that confer structural integrity to the web of viscose fibers. Preferred polyolefin fibers of the absorbent non-woven layer is selected from the group comprising polyester fibers, polypropylene fibers and bico fibers, or combinations thereof. Bico fibers (bicomponent fibers) are conjugate fibers made of two different types of polymers. A bico fiber can e.g. be made by co-extruding one polymer outside a strand of another polymer. A common bico fibers is e.g. composed of a low melt thermoplastic polyethylene extruded around a strand of polypropylene or polyester. An alternative bico fiber strand is made by spinning two polymeric components separately and joining them in a simultaneous process from one common spinneret. When the outer fibers of the bico fiber are heated they melt and bond the other fiber constituents of the absorbent non-woven layer together.

Specific examples of suitable absorbent components include but are not limited to one or more of carboxymethyl cellulose fibers and calcium alginate fibers, or combinations thereof.

In addition or in the alternative the absorbent non-woven layer can be coated with powdered superabsorbent polymer.

An example of a suitable superabsorbent fiber is cross-linked acrylate copolymer fiber.

In a particular simple and inexpensive embodiment the absorbent non-woven layer may be composed substantially exclusively of viscose fibers.

The composition the fibers in the absorbent non-woven layer can be tailored in numerous ways. The preferred ratio of viscose fibers to polyolefin fibers in the absorbent non-woven layer is 100:0 wt% based on the total weight of viscose fibers and polyolefin fibers, alternatively 75:25 wt% based on the total weight of viscose fibers and polyolefin fibers, alternatively 50:50 wt% based on the total weight of viscose fibers and polyolefin fibers, alternatively 25:75 wt% based on the total weight of viscose fibers and polyolefin fibers, and most preferably 75:25 wt% based on the total weight of viscose fibers and polyolefin fibers. The appropriate ratio depends a.o. on intended absorption capacity according to a given volume of exudates for which the dressing is intended, level of integrity of laminate and coherence of the absorbent non-woven layer.

The superabsorbent fibers and/or gel-forming fibres of the absorbent non-woven layer may be provided in an amount that provides the wound dressing laminate with an absorption capacity of at least 20, more preferred at least 25, even more preferred at least about 30, and most preferred up to 40 g fluid per g fiber(s).

The preferred layer impregnated with antimicrobial agent may be selected from the group comprising a knitted fabric, a net or mesh, all of which being apertured structures able to retain large doses of antimicrobial agent and release said antimicrobial agent in a controlled sustained manner. Thus the wound dressing laminate can be loaded with higher doses of antimicrobial agent than known similar dressing materials at no increased risk of cytotoxic side effects.

In an advantageous embodiment the layer impregnated with antimicrobial agent is a viscose layer, preferably a knitted viscose fabric.

The antimicrobial agent of the impregnated layer may be dispersed or suspended in an ointment, wherein the antimicrobial agent represents 1 - 10 wt% of the total weight of the ointment. Such ointment suspension is used as the impregnation solution, which is spread over the layer to be impregnated. The antimicrobial agent containing ointment not only settles on the fiber structure of the layer to be impregnated, it also flows into the apertures and voids of said layer, where it settles to make chemical depositories, wherefrom antimicrobial agents can release upon contact with moisture, such as exudates.

The antimicrobial agent of the impregnated layer is povidone-iodine, and in a preferred embodiment in form of a polyglycol/povidone-iodine ointment, wherein the povidone-iodine represent 1 - 10 wt% povidone-iodine of the total weight if the ointment, however polyglocol may serve as the solvent or suspension agent for any antimicrobial agent suited for the wound dressing laminate of the present invention
The wound dressing laminate may include a secondary absorbent layer laminated to the absorbent non-woven layer to further ensure high absorbent capacity. The non-woven layer may have same or different composition as the absorbent non-woven layer, and be made the same way or in a different way than the absorbent non-woven layer. Preferable the secondary absorbent layer is also a non-woven layer, optionally of less thickness than the absorbent non-woven layer.

The invention is also directed to a method as according to claim 8 of manufacturing a wound dressing laminate comprising a layer impregnated with antimicrobial agent, which method comprises the steps of
- providing the layer impregnated with an antimicrobial agent,
- providing an apertured wound-contacting layer adjacent one side of the layer impregnated with the antimicrobial agent,
- providing an absorbent non-woven layer adjacent the opposite side of the layer impregnated with the antimicrobial agent, and
- laminating the layers together.

Since many antimicrobial agents, in particular povidone-iodine ointments, are very heat sensitive, it has hitherto not been possible nor contemplated to laminate an apertured wound-contacting layer on a layer impregnated with such antimicrobial agent. Rather a wound-contacting layer is dispensed with and separate secondary bandages used.

The inventors of the present invention has now been successful in bonding a layer impregnated with antimicrobial agent, e.g. a fabric impregnated with povidone-iodine ointment, to an absorbent non-woven layer using a first mechanical bonding step, thereby obtaining a laminate being a coherent double-ply structure without using heat application. Needle punching is a cold mechanical bonding process that is particular suited for the first mechanical bonding step.

In order to obtain the final wound dressing laminate the apertured wound-contacting layer is also laminated to the layer impregnated with antimicrobial agent at the side free of absorbent non-woven layer to obtain a coherent triple-ply structure using a second mechanical bonding step. The second mechanical bonding step may be performed prior to the first mechanical bonding step or after said mechanical bonding step.

The second mechanical bonding step may be calendaring or needle punching whatever is the most appropriate given the selection of fibers and choice of layers and antimicrobial agent. The person skilled in the art is able to establish by test and trial the most efficient second mechanical step.

The layer impregnated with antimicrobial agent may be manufactured by passing a layer of non-impregnated fabric through a bath with a mixture of antimicrobial agent and allowing the antimicrobial agent to deposit on the impregnated fabric. The bath may be heated to a temperature selected in view of the heat sensitivity of the antimicrobial agent and associated solvent or suspension agent. The temperature of the bath is considerable lower than the temperature that would have been needed for heat lamination so the temperature of the bath has no negative impact on the antimicrobial activity of the antimicrobial agent.

The antimicrobial agent is povidone-iodine and the layer impregnated with povidone-iodine is in an advantageous embodiment manufactured by passing a layer of non-impregnated fabric through a heated bath with a mixture of polyethylen glycol and povidone-iodine and allowing the heated impregnated fabric to cool.

The absorbent non-woven layer may be manufactured by
a) providing a blend of one or more fiber components selected from the group comprising viscose fibers, polyolefin fibers and/or superabsorbent fibers,
b) carding the fiber blend to obtain a web of mixed fiber components,
c) passing said web through a cross lapper after carding the fiber blend in step b),
d) needle punching the web from at least one side, and
e) calendering the needle punched web to obtain the absorbent non-woven layer laminate.

Carding is used to mechanically break up locks and clumps of fiber and then aligns the individual fibers so that they are more or less parallel with each in a still coherent non-woven web. In the cross-lapper the non-woven fiber web is folded or laid down several times to achieve a web of suitable thickness, which web then is needle punched. The barbed needles carries tufts of the web's fibers crosswise the web to make it vertically coherent. Finally, the web is calendered to obtain further bonding of fibers of the web.

The method may further comprise the step of
f) coating the web of absorbent non-woven layer with powders coated with superabsorbent polymer.

The method may further comprise the step of laminating the absorbent non-woven layer side of the wound dressing laminate to a layer of secondary absorbent dressing. To achieve even further improved absorbent capacity directed away from the wound the secondary layer of absorbent dressing may have a higher absorptive capacity than the absorbent non-woven layer.

Examples of suitable secondary layers of absorbent dressing includes a foam layer, preferably of polyurethane.

The invention also relates to a wound dressing that includes a pad of the wound dressing laminate as described above. To prevent leakage of antimicrobial agent the periphery of the wound dressing may be sealed using a mechanical sealing step, such as ultrasonic welding or heat sealing.

An island pad wound dressing made in accordance with the invention can be made in many sizes and with different loads of antimicrobial agent to be used in treatment of exudates wounds.

The invention will now be described by way of exemplary embodiments with reference to the accompanying drawing, in which
fig. 1 shows, seen in perspective, an exploded view of a preferred embodiment of a wound dressing according to the present invention,
fig. 2 shows, seen in perspective oblique from the side facing the skin in use, the wound dressing seen in fig. 1 in assembled state,
fig. 3 shows schematically a production line for manufacturing the layer impregnated with antimicrobial agent,
fig. 4 shows schematically a production line for manufacturing the absorbent non-woven layer,
fig. 5 shows schematically a lamination method of a three-layered wound dressing laminate according to the present invention, and
fig. 6 shows a production line for coating a fiber web with a super absorbent powder.

The wound dressing according to the present invention is described in more detail below by way of an exemplary design. Although the wound care dressing are shown in the figures to be square with smooth corners, the figures should not be construed to limit the design and outline of the wound care dressings according to the invention. Many other sizes and shapes are contemplated within the scope of the present invention.

The thicknesses of the various layers are indicated in the figures for illustrative purposes and by way of example. Also by way of example the apertured wound-contacting layers are shown with evenly distributed identical apertures, and patterns and signatures used in exemplary manner to indicated the nature of a layer.

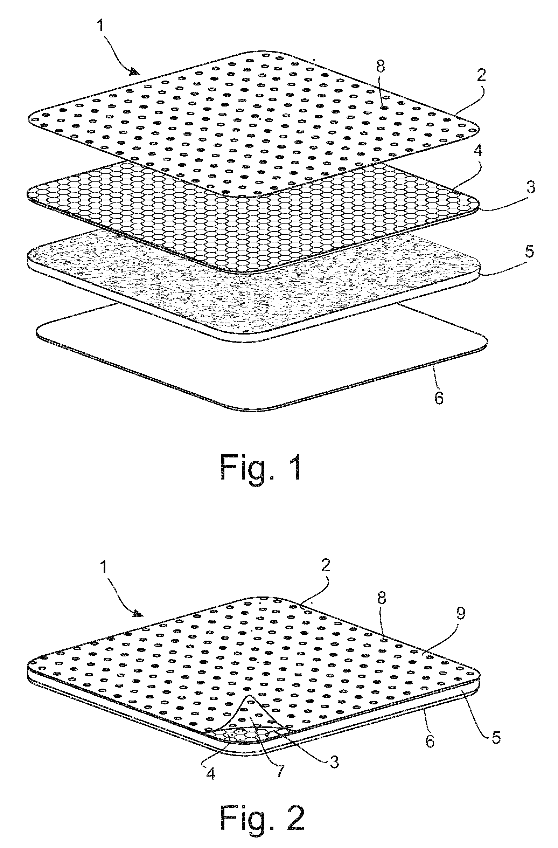

Fig. 1 shows an exploded view of an embodiment of a wound dressing 1 consisting of an apertured wound-contacting film layer 2, a layer 3 impregnated with an antimicrobial agent 4, an absorbent non-woven layer 5 and a secondary absorbent layer 6.

The four layers 2, 3 ,5, and 6 are laminated together to obtain the coherent laminated wound dressing seen in fig. 2 wherein a flap 7 of the apertured wound-contacting layer 2 is lifted for illustrative purposes to visualize the layer 3 impregnated with antimicrobial agent 4 being disposed between the apertured wound-contacting layer 2 and the absorbent non-woven layer 5. Emphasis is made that for the final wound dressing 1 no flap 7 is left free. The antimicrobial agent 4 is dispersed all over the impregnated layer 3 and is able to pass via apertures 8 or equivalent through-going passages or channels to the outside 9 of the apertured wound-contacting layer 2.

A final laminate wound dressing may for example include a 0.135 mm polyethylene (PE) net, a 0.5 mm knitted viscose fabric impregnated with a 10% povidone-iodine/polyglycol ointment, a 0.5 mm absorbent non-woven felt layer to obtain a wound dressing with a total thickness of 1.135 mm when the three layers are laminated together. In case a secondary absorbent layer is included a secondary absorbent non-woven felt might be 0.15 mm to obtain a four-layered laminated wound dressing with a total thickness of 1.150 mm. The knitted fabric constitutes a strong coherent structure that is unlikely to disintegrate during impregnation. So knitted fabrics are good carrier supports for the antimicrobial agent.

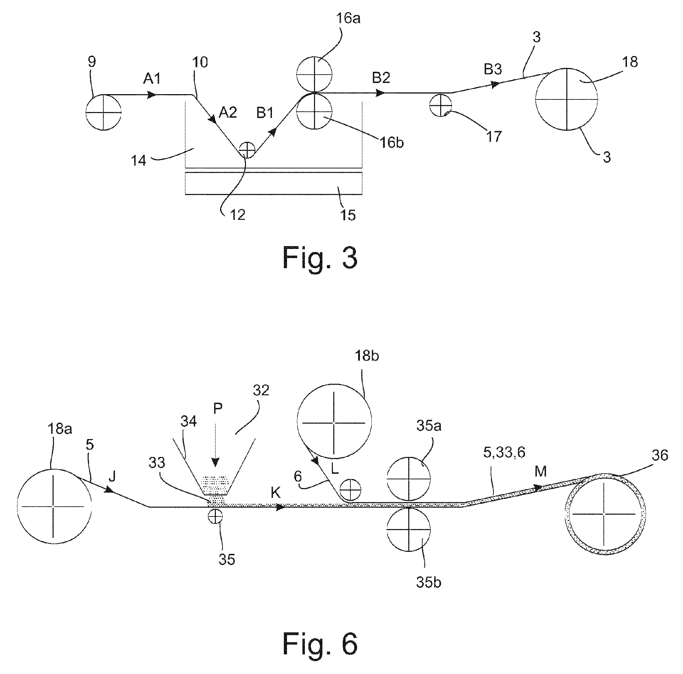

Preparation of the individual layers of the wound dressing laminate and manufacturing of the laminate according to the present invention will be described below with reference to figs. 3-6. The apertured wound-contacting layer is for sake of simplicity named a net, the layer impregnated with antimicrobial agent is named a fabric, the antimicrobial agent named PVP-I and the absorbent non-woven web named felt. These names should not be seen as limiting the scope of the present invention but help to make the below description of the method steps easier to understand. Thus all alternative named and discusses in the preceding description can of course be made and prepared in similar methods steps.

Fig. 3 shows schematically a production line for manufacturing the layer impregnated 3 with antimicrobial agent 4, thus the fabric impregnated with PVP-I containing ointment.

From rewinder 9 the fabric 10, with no antimicrobial agent, is conveyed in the direction of arrows A1 and A2 below conveyer roll 12 located in dipping bath 13 with the solution of PVP-I and polyglycol ointment 14 that is heated by means of heater 15. As indicated by the arrows B1 and B2 the wet impregnated fabric 10 is then passing through squeezing rollers 16a,16b to cool while being conveyed by means of conveyer roll 17, as indicated with arrow B3, and wound on fabric winder 18 for later use in dressing making. The gap between squeezing rollers 16a,16b is adjustable according to the required ointment 14 coat weight, thus the squeezing rollers 16a,16b remove excess ointment so that ointment coat weight can be adjusted.

Optionally conveyer roll 17 is cooled, or the conveyer roll 17 is placed in a cooling zone. The appropriate temperature of the dipping bath is selected in dependency on the temperature sensivity of the antimicrobial agent and the temperature by which the solvent or suspension agent is liquid. The fabric 10 is conveyed with a speed selected to allow an intended concentration of antimicrobial agent to be deposited on the fabric and the production line adjusted to allow this antimicrobial agent to deposit on the fabric before being wound on fabric winder 18.

The so obtained impregnated layer 3 can be stored on winders until further use when manufacturing the wound dressing laminate.

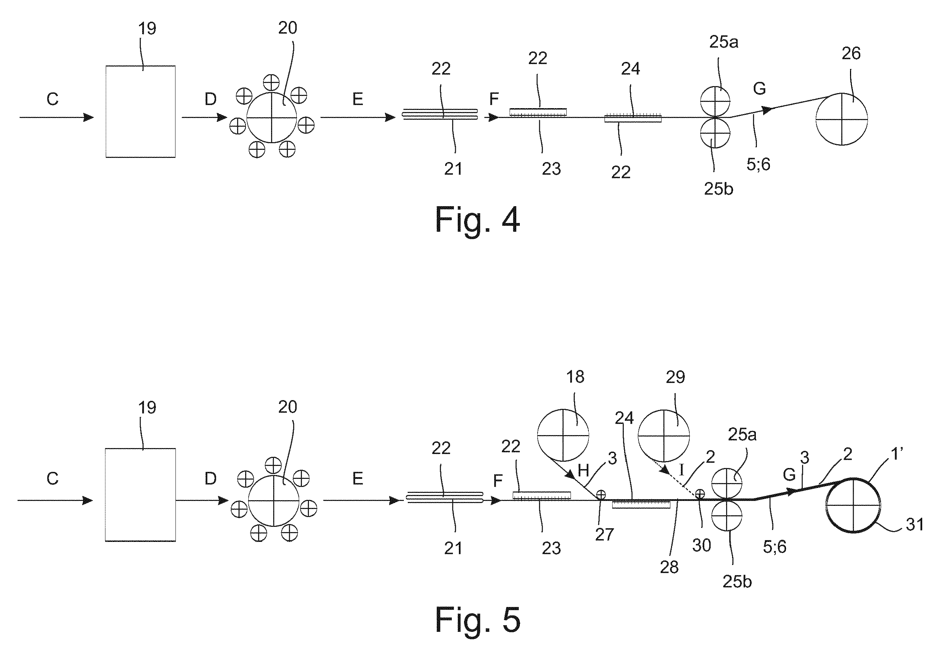

Fig. 4 shows schematically a production line for manufacturing an absorbent non-woven layer 5 or a secondary absorbent layer 6, in the following felt 5;6.

As indicated by arrow C a suitable selection of fiber components for producing the felt 5;6 is supplied to fiber opener 19. The fiber components can for example be a blend M1 consisting of viscose fibers and polyolefin fibers or a blend M2 consisting of viscose fibers, polyolefin fibers and superabsorbent fibres. After being opened in fiber opener 19, the blend of fiber components is passed via carder 20, as indicated with arrow D, to cross-lapper 21, as indicated with arrow E, to obtain loose felt layer 22 of increased thickness. Felt layer 22 is then conveyed in the direction of arrow F, first to a first needle punching station 23 where felt layer 22 is needle punched mechanically from below, and next to a second needle punching station 24 where felt layer 22 is needle punched mechanically from the top to achieve the desired coherent non-woven felt 5;6 which is conveyed via calender rollers 25a,25b to felt winder 26, as indicated by arrow G.

The so obtained felt 5;6 can be stored on winders until further use when manufacturing the wound dressing laminate.

When the wound dressing laminated is made the apertured wound-contacting layer, the impregnated layer and the non-absorbents layers are supplied in respective order from each their rewinder and laminated together using a mechanical lamination procedure, e.g. needle punching.

Fig. 5 shows schematically a continuous manufacturing method of a three-layered wound dressing laminate according to the present invention. The absorbent non-woven layer is made in the same manufacturing method and until the first part of the method from the opener to the first needle punching station corresponds to the method described in fig. 4 and for like parts same reference numerals are used.

In the continuous manufacturing method the impregnated fabric 3 is supplied from winder 18 between first needle punching station 23 and the second needle punching station 23, as indicated by arrow H. By means of roller 27 the impregnated fabric 3 is brought in contact with the felt 22 already needle punched from below at the first needle punching station and conveyed to the second needle punching station where the impregnated fabric 3 is laminated to the top of the felt 22 by needle punching again from the top to obtain a two-layered intermediate laminate 28. As indicated by arrow I a rewinder 29 provides a layer of apertured wound-contacting net 2 via roller 30 on top of the two-layered intermediate laminate 28 to obtain three layers to pass through calendar rollers 25a,25b to obtain the three layered wound dressing laminate 1' rolled on winder 31 for later manufacturing of wound dressings 1, e.g. by die cutting.

Fig. 6 shows a production line for coating a fiber web with a super absorbent powder.

An absorbent layer 5 of blend M1 manufactured as described for fig. 4 is rewinded a first winder 18a, as indicated by arrow J, and conveyed to super absorbent powder coating station 32, where the super absorbent powder 33 falls from reservoir 34, as indicated by arrow P, onto first absorbent non-woven layer 5, and is conveyed by means of roller 35 in the direction indicated by arrow K below a second winder 18b with secondary absorbent non-woven layer 6 of similar or different structure and composition as the first absorbent non-woven layer 5, as indicated by arrow L. The sandwich of first absorbent non-woven layer 5, superabsorbent powder 33 and secondary absorbent non-woven layer 6 are then passed through calendar rollers 35a,35b before being conveyed to powder coated felt winder 36 as indicated by arrow M.

### Test Examples

### Example 1

A quantitative study of the antimicrobial properties of the antimicrobial wound dressing laminate according to the present invention and a corresponding control dressing without povidone-iodine when challenged with weight microorganisms strains

Laminate wound dressings (PharmaPOVI®) were manufactured in accordance with the invention and tested at Bioscience Laboratories, INC, 300 N, Wilson Avenue, Bozeman, Montana 59716. The wound dressings were made January 2012 and tested same month. The sample wound dressing laminate consisted of a 0.135 mm thick polyethylene wound-contacting net layer, a 0.5 mm thick layer of a knitted viscose fabric impregnated with polyglycol based ointment containing 10% PVP-Iodine, and a 0.5 mm thick non-woven felt layer made of a blend of viscose fibers and bico fibers.

The antimicrobial wound dressing above (Test sample) and a non-impregnated control material (Control) were tested. Prior to testing, three lots of samples were aseptically cut into swatches, each approximately 2.5 cm x 2.5 cm in size. Each swatch of each material were contaminated with a broth culture of a challenge strain, to produce an initial inoculum level of approximately 10⁶ Colony-Forming Units (CFU) per swatch. One swatch of each of the Test samples and one swatch of the Controls were challenged versus each microorganism strain using a contact/exposure time of 2 hours; a second swatch of each of the lots of the Test samples and the Controls was challenged versus each microorganism strain using a contact/exposure time of 24 hours. Insofar as it was possible, the contaminated swatches of each material were maintained at 35 ± 2°C for the duration of each contact/exposure time. Following each exposure, the viable microorganisms was recovered from each swatch by rinsing, diluting, and plating aliquots, in duplicate. The log₁₀ microbial population of each challenge species (CFU/swatch) recovered from each material were determined for each exposure/contact time by pour- or spread-plated prepared dilutions on Petri plates, in duplicates, using the conditions indicated in Reference Table. Testing were conducted using a modification of AATCC (American Association of Textile Chemists and Colorists) Test Method 100-2004, *Antibacterial Finishes on Textile Materials, Assessment of.*

### Challenge microorganisms strains

1. *Aspergillus brasiliensis* (ATCC#16404)
   Prior to testing, inocula from a lyophilized vial was suspended in 0.9 % Sodium Chloride Irrigation, USP (SCI), inoculated onto the surface of Potato Dextrose Agar (PDA) contained in Petri plates, and incubated at 25°C ± 2°C for 10 to 14 days, or until sufficient growth is observed. This produced lawns of the challenged fungus on the surface of the agar plates, and conidia from these was used to prepare useful challenge suspensions. Once sufficient growth was observed, the culture plates were sealed and stored at 2°C - 8°C, if needed, for up to 4 weeks prior to harvesting the conidia for use in testing.
2. *Candida albicans* (ATCC#10231)
   Approximately 48 hours prior to testing a sterile tube containing Sabourand Dextrose Broth (SDB) was inoculated from a lyophilized vial. The broth culture was incubated at 30°C ± 2°C for approximately 24 hours or until sufficient growth was observed. Approximately 24 hours to testing, the broth culture was inoculated onto the surface of the Sabourand Dextrose Agar (SDA) contained in Petri plates, and incubated at 30°C ± 2°C for 10 to 14 days, or until sufficient growth is observed. This produced lawns of the yeast on the surface of the agar plates, and growth from these was used to prepare useful challenge suspension. The purity of the culture was verified by preparing an isolation streak of the culture on SDA, and incubating appropriately.
3. *Entero faecalis* VRE (ATCC#51299), 4. *Escherichia Coli* (ATCC#11229), 5. *Klebsiella pneumonia pneumonia* (ATCC#10031), 6. *Pseudomonas aeruginosa* (ATCC#27853), 7. *Staphylococcus aureus aureus* (ATCC#6538), 8. *Staphylococcus aureus aureus* MRSA (ATCC#33591), (VRE: Vancomycin-Resistant *Enterococcus;* MRSA: Methicilli-Resistant *Staphylococcus aureus*).

Approximately 48 hours prior to testing separate sterile tubes containing Tryptic Soy Broth (TSB) was inoculated from lyophilized vials. The broth cultures was incubated at 35°C ± 2°C for approximately 24 hours or until sufficient growth was observed. Approximately 24 hours to testing, the broth cultures were inoculated onto the surface of Tryptic Soy Broth (TSB) contained in Petri plates, and incubated at 35°C ± 2°C. This produced lawns of the bacteria on the surface of the agar plates, and growth from these was used to prepare useful challenge suspensions. The purity of each culture was verified by preparing an isolation streak of the culture on SDA, and incubating appropriately.

**REFERENCE TABLE**

| No | Challenge species | ATCC# | Incubation time | Incubation temperature | Media (Plating method) |
|---|---|---|---|---|---|
| 1 | *Aspergillus brasiliensis* | 16404 | 2 to 14 days | 25°C ± 2°C | SDB/PDA/SDA+ (Spread-plates) |
| 2 | *Candida albicans* | 10231 | 24 to 72 hours | 30°C ± 2°C | SDB/SDA/SDA+ (Pour-plates) |
| 3 | *Entero faecalis* VRE | 51299 | 24 to 72 hours | 35°C ± 2°C | TSB/TSA/TSA+ (Pour-plates) |
| 4 | *Escherichia Coli* | 11229 | 24 to 72 hours | 35°C ± 2°C | TSB/TSA/TSA+ (Pour-plates) |
| 5 | *Klebsiella pneumonia pneumonia* | 10031 | 24 to 72 hours | 35°C ± 2°C | TSB/TSA/TSA+ (Pour-plates) |
| 6 | *Pseudomonas aeruginosa* | 27853 | 24 to 72 hours | 35°C ± 2°C | TSB/TSA/TSA+ (Pour-plates) |
| 7 | *Staphylococcus aureus aureus* | 6538 | 24 to 72 hours | 35°C ± 2°C | TSB/TSA/TSA+ (Pour-plates) |
| 8 | *Staphylococcus aureus aureus* MRSA | 33591 | 24 to 72 hours | 35°C ± 2°C | TSB/TSA/TSA+ (Pour-plates) |

| | | | | | |
|---|---|---|---|---|---|
| VRE: Vancomycin-Resistant *Enterococcusl* MRSA: Methicillin-Resistant *Staphylococcus aureus aureus* Note: Incubation times are nominal, but in practise incubation time will continue until growth is observed. | | | | | |

The results of the quantitative study of the antimicrobial properties of the wound dressing laminate according to the present invention are indicated in TABLE 1 - 4 below.

**TABLE 1: ANTIMICROBIAL EFFICACY EVALUATION - TEST RESULTS**

| Test Material: PharmaPOVI Pad; 10% PVP Iodine Lot Number 1/2012 Non-aged Sample | | | | | | |
|---|---|---|---|---|---|---|
| No. | Challenge Microorganism (ATCC Number) | Inoculum Level (CFU/swatch) | Exposure Time | Post-Exposure Population (CFU/swatch) | Percent Reduction | Log₁₀ Reduction |
| 1 | *Candida albicans* (ATCC #10231) | 4.20 x 10⁶ | 2 hours | < 2.00 x 10¹ | 99.9995% | 5.3222 |
| | | | 24 hours | < 2.00 x 10¹ | 99.9995% | 5.3222 |
| 2 | *Enterococcus faecalis* VRE (ATCC #51299) | 1.40 x 10⁷ | 2 hours | < 2.00 x 10¹ | 99.9999% | 5.8451 |
| | | | 24 hours | < 2.00 x 10¹ | 99.9999% | 5.8451 |
| 3 | *Escherichia coli* (ATCC #11229) | 6.850 x 10⁶ | 2 hours | < 2,00 x 10¹ | 99.9997% | 5.5347 |
| | | | 24 hours | < 2.00 x 10¹ | 99.9997% | 5.5347 |
| 4 | *Klebsiella pneumoniae pneumoniae* (ATCC #10031) | 6.8250 x 10⁶ | 2 hours | < 2.00 x 10¹ | 99.9997% | 5.5331 |
| | | | 24 hours | < 2.00 x 10¹ | 99.9997% | 5.5331 |
| 5 | *Pseudomonas aeruginosa* (ATCC #27853) | 6.6250x10⁶ | 2 hours | < 2.00 x 10¹ | 99.9997% | 5.5202 |
| | | | 24 hours | < 2.00 x 10¹ | 99.9997% | 5.5202 |
| 6 | *Staphylococcus aureus aureus* (ATCC #6538) | 8.00 x 10⁶ | 2 hours | < 2.00 x 10¹ | 99.9998% | 5.6021 |
| | | | 24 hours | < 2.00 x 10¹ | 99.9998% | 5.6201 |
| 7 | *Staphylococcus aureus aureus* MRSA (ATCC #33591) | 8.20 x 10⁶ | 2 hours | < 2.00 x 10¹ | 99.9998% | 5.6128 |
| | | | 24 hours | < 2.00 x 10¹ | 99.9998% | 5.6128 |

| | | | | | | |
|---|---|---|---|---|---|---|
| VRE = Vancomycin-Resistant *Enterococcus* MRSA = Methicillin-Resistant *Staphylococcus aureus* | | | | | | |

**TABLE 2: ANTIMICROBIAL EFFICACY EVALUATION - TEST RESULT**

| Test Material: PharmaPOVI Pad; 10% PVP Iodine Lot Number 2/2012 Non-aged Sample | | | | | | |
|---|---|---|---|---|---|---|
| No. | Challenge Microorganism (ATCC Number) | Inoculum Level (CFU/swatch) | Exposure Time | Post-Exposure Population (CFU/swatch) | Percent Reduction | Log₁₀ Reduction |
| 1 | *Candida albicans* (AATCC#10231) | 4.20 x 10⁶ | 2 hours | < 2.00 x 10¹ | 99.9995% | 5.3222 |
| | | | 24 hours | < 2.00 x 10¹ | 99.9995% | 5.3222 |
| 2 | *Enterococcus faecalis* VRE (ATCC #51299) | 1.40 x 10⁷ | 2 hours | < 2.00 x 10¹ | 99.9999% | 5.8451 |
| | | | 24 hours | < 2.00 x 10¹ | 99.9999% | 5.8451 |
| 3 | *Escherichia coli* (ATCC #11229) | 6.850 x 10⁶ | 2 hours | < 2.00 x 10¹ | 99.9997% | 5.5347 |
| | | | 24 hours | < 2.00 x 10¹ | 99.9997% | 5.5347 |
| 4 | *Klebsiella pneumoniae pneumoniae* (ATCC #10031) | 6.8250 x 10⁶ | 2 hours | < 2.00 x 10¹ | 99.9997% | 5.5331 |
| | | | 24 hours | < 2.00 x 10¹ | 99.9997% | 5.5331 |
| 5 | *Pseudomonas aeruginosa* (ATCC #27853) | 6.6250 x 10⁶ | 2 hours | < 2.00 x 10¹ | 99.9997% | 5.5202 |
| | | | 24 hours | < 2.00 x 10¹ | 99.9997% | 5.5202 |
| 6 | *Staphylacoccus aureus aureus* (ATCC #6538) | 8.00 x 10⁶ | 2 hours | < 2.00 x 10¹ | 99.9998% | 5.6021 |
| | | | 24 hours | < 2.00 x 10¹ | 99.9998% | 5.6201 |
| 7 | *Staphylococcus aureus aureus* MRSA (ATCC #33591) | 8.20 x 10⁶ | 2 hours | < 2.00 x 10¹ | 99.9998% | 5.6128 |
| | | | 24 hours | < 2.00 x 10¹ | 99.9998% | 5.6128 |

| | | | | | | |
|---|---|---|---|---|---|---|
| VRE = Vancomycin-Resistant *Enterococcus* MRSA = Methicillin-Resistant *Staphylococcus aureus* | | | | | | |

**TABLE 3: ANTIMICROBIAL EFFICACY EVALUATION - TEST RESULTS**

| Test Material: PharmaPOVI Pad; 10% PVP Iodine Lot Number 3/2012 Non-aged Sample | | | | | | |
|---|---|---|---|---|---|---|
| No. | Challenge Microorganism (ATCC Number) | Inoculum Level (CPU/swatch) | Exposure Time | Post-Exposure Population (CPU/swatch) | Percent Reduction | Log₁₀ Reduction |
| 1 | *Candida albicans* (ATCC #110231) | 4.20 x 10⁶ | 2 hours | < 2.00 x 10¹ | 99.9995% | 5.3222 |
| | | | 24 hours | < 2.00 x 10¹ | 99.9995% | 5.3222 |
| 2 | *Enterococcus faecalis* VRE (ATCC #51299) | 1.40 x 10⁷ | 2 hours | < 2.00 x 10¹ | 99.9999% | 5.8451 |
| | | | 24 hours | < 2.00 x 10¹ | 99.9999% | 5.8451 |
| 3 | *Escherichia coli* (ATCC #11229) | 6.850 x 10⁶ | 2 hours | < 2.00 x 10¹ | 99.9997% | 5.5347 |
| | | | 24 hours | < 2.00 x 10¹ | 99.9997% | 5.5347 |
| 4 | *Klebsiella pneumoniae pneumoniae* (ATCC # 10031) | 6.8250 x 10⁶ | 2 hours | < 2.00 x 10¹ | 99.9997% | 5.5331 |
| | | | 24 hours | < 2.00 x 10¹ | 99.9997% | 5.5331 |
| 5 | *Pseudomonas aeruginosa* (ATCC #27853) | 6.6250 x 10⁶ | 2 hours | < 2.00 x 10¹ | 99.9997% | 5.5202 |
| | | | 24 hours | < 2.00 x 10¹ | 99.9997% | 5.5202 |
| 6 | *Staphylococcus aureus aureus* (ATCC #6538) | 8.00 x 10⁶ | 2 hours | < 2.00 x 10¹ | 99.9998% | 5.6021 |
| | | | 24 hours | < 2.00 x 10¹ | 99.9998% | 5.6201 |
| 7 | *Staphylococcus aureus aureus* MRSA (ATCC #33591) | 8.20 x 10⁶ | 2 hours | < 2.00 x 10¹ | 99.9998% | 5.6128 |
| | | | 24 hours | < 2.00 x 10¹ | 99.9998% | 5.6128 |

| | | | | | | |
|---|---|---|---|---|---|---|
| VRE = Vancomycin-Resistant *Enterococcus* MRSA = Methicillin-Resistant *Staphylococcus aureus* | | | | | | |

**TABLE 4: ANTIMICROBIAL EFFICACY EVALUATION - TEST RESULTS**

| Control Material: PharmaPOVI untreated control sample Lot Number Sample Non-aged Sample | | | | | | |
|---|---|---|---|---|---|---|
| No. | Challenge Microorganism (ATCC Number) | Inoculum Level (CFU/swatch) | Exposure Time | Post-Exposure Population (CFU/swatch) | Percent Reduction | Log₁₀ Reduction or *Log₁₀ Increase* |
| 1 | *Candida albicans* (AATCC #10231) | 4.20 x 10⁶ | 2 hours | 3.850 x 10⁶ | 8.3333% | 0.0377 Reduction |
| | | | 24 hours | 6.70 x 10⁷ | NC | *1.2029 Increase* |
| 2 | *Enterucoccus faecalis* VRE (ATCC #51299) | 1.40 x 10⁻⁷ | 2 hours | 2.020 x 10⁷ | NC | *0.1593 Increase* |
| | | | 24 hours | 3.30 x 10⁵ | 97.6429% | 1.6276 Reduction |
| 3 | *Escherichia coli* (ATCC #11229) | 6.850 x 10⁶ | 2 hours | 3.00 x 10⁶ | 56.2044% | 0.3586 Reduction |
| | | | 24 hours | 1.530 x 10⁷ | NC | *0.3490 Increase* |
| 4 | *Klebsiella pneumoniae pneumoniae* (ATCC #10031) | 6.8250 x 10⁶ | 2 hours | 9.80 x 10⁵ | 85.6410% | 0.8429 Reduction |
| | | | 24 hours | < 1.00 x 10³ | 99.9853% | 3.8341 Reduction |
| 5 | *Pseudomonas aeruginosa* (ATCC #27853) | 6.6250 x 10⁶ | 2 hours | 1.1850 x 10⁶ | 83.1132% | 0.7475 Reduction |
| | | | 24 hours | < 1.00 x 10³ | 99.9849% | 3.8212 Reduction |
| 6 | *Staphylococcus aureus aureus* (ATCC #6538) | 8.00 x 10⁶ | 2 hours | 6.650 x 10⁶ | 16.8750% | 0.0803 Reduction |
| | | | 24 hours | 1.6450 x 10⁸ | NC | *1.3131 Increase* |
| 7 | *Staphylococcus aureus aureus* MRSA (ATCC #33591) | 8.20 x 10⁶ | 2 hours | 1.0750 x 10⁷ | NC | *0.1176 Increase* |
| | | | 24 hours | 7.650 x 10⁸ | NC | *1.969 Increase* |

| | | | | | | |
|---|---|---|---|---|---|---|
| VRE = Vancomycin-Resistant *Enterococcus* MRSA = Methicillin-Resistant *Staphylococcus aureus* NC = Reduction Not Calculated | | | | | | |

### Conclusion

The wound dressing according to the present invention is substantially 100 % effective against the tested bacteria and fungi.

The study confirms that the inventive wound dressing laminate, that does not adhere to the wound dressing bed, can release the antimicrobial agent povidone-iodine despite the layers of the laminate are mechanically laminated to each other. The study confirms that the mechanical laminating procedures have no negative impact on the antibacterial and antifungal activity of povidone-iodine.

### Example 2

### Determination of the virucidal properties of the antimicrobial wound dressing laminate according to the present invention and a corresponding control dressing without povidone-iodine when challenged with viral strains

The study is designed to evaluate the same wound dressing laminates (PharmaPOVI®) as above for virucidal efficacy. Three non-aged lots of samples of the wound dressing laminate defined in Example 1 were tested versus challenge viral strains: Human Immunodeficiency Virus (HIV-1, strain Mn; ZeptoMetrix # 0810027CF) and Bovine Viral Diarrhea Virus (BVDV, ATCC #VR-534), a surrogate for HCV.

Three lots of each wound dressing laminate were cut to swatces of 3.0cm x 3.0cm and tested for virucidal efficacy when compared to that of similar sized swatches of controls without povidine-iodine (Untreated). The test viruses were inoculated onto the test samples and controls and eluted following a 2-hour and 24-hour incubation and exposure time at 37°C ± 2°C in a humidified CO₂ incubator. The surviving viruses were serially diluted and plated onto susceptible cells. Each dilution were plated in four replicates. Viral titers were expressed as log₁₀ of the 50% titration end point for infectivity - 50% Tissue Culture Infectious Dose [TCID₅₀] (Spearman-Kärber Method). The log₁₀ and percent reductions in numbers of each strain produced by the antimicrobial-treated samples were calculated by comparison with the numbers of each strain recovered from the control, as well as with the initial viral population used for contamination. The study used a modification of AATCC Test Method 100-2004 *"Antibacterial Finishes on Textile Materials: Assessment of".*

### Host cell preparation

Bovine Turbinate (BT) cells (ATCC #CRL-1390) maintained as monolayers in disposable cell culture labware and C8166 (Human T cell leukemia [ECACC #88051601]) maintained as a suspension culture in sufficient cell population were used for testing. The Growth Medium (GM) was Dulbecco's Modified Eagle Medium (DMEM) with appropriate supplements and 10% Horse Serum for BT cells. DMEM with appropriate supplements were used as Maintenance Medium (MM). GM and MM for C8166 cells were 1X RPMI 1640 with appropriate supplements (FBS, antibiotics, L-glutamine). Prior to testing, the GM will be replaced by MM.

### Test virus preparation

Prior to use, HIV-1 strain Mn was propagated in C8166 cells and used for testing when at least 50% of infected cells appeared syncytial. BVDV strain NADL was propagated in BT cells and used for testing when virus Cytopathic Effect (CPE) reaches its maximum.

The results of the virucidal properties of the wound dressing laminate according to the present invention are indicated in TABLE 5 - 18 below.

**TABLE 5**

| Test Product: PharmaPOVI Lot # 1/2012, 2/2012, 3/2012 | | | | | | |
|---|---|---|---|---|---|---|
| Control Product: PharmaPOVI Untreated | | | | | | |
| Virus / Strain: BVDV/ NADL ATCC #VR-534 | | | | | | |
| Host Cell Line: BT Host Cell Line ATCC #CRL-1390 | | | | | | |
| Volume Plated per Well: 1.0 mL | | | | | | |
| Exposure: 2 hours | | | | | | |
| **Dilution (-Log₁₀)** | **Control Product** | | | **Test Product** | | |
| | **Rep 1** | **Rep 2** | **Rep 3** | **Lot# 1/2012** | **Lot# 2/2012** | **Lot# 3/2012** |
| -2 | ++++ | ++++ | ++++ | 0000 | 0000 | 0000 |
| -3 | ++++ | ++++ | ++++ | 0000 | 0000 | 0000 |
| -4 | ++++ | ++++ | ++++ | 0000 | 0000 | 0000 |
| -5 | ++0+ | ++++ | ++++ | 0000 | 0000 | 0000 |
| -6 | +0+0 | 000+ | +0+0 | 0000 | 0000 | 0000 |
| TCID₅₀ (Log₁₀) | 5.75 | 5.75 | 6.00 | ·1.50 | ·1.50 | ·1.50 |
| Average TCID₅₀ (Log₁₀) | 5.83 | | | ·1.50 | | |
| Log₁₀ Reduction | NA | | | **·4.33** | **·4.33** | **·4.33** |
| Average Log₁₀ Reduction | NA | | | **·4.33** | | |
| Average Percent Reduction | NA | | | **99.9998%** | | |

| | | | | | | |
|---|---|---|---|---|---|---|
| **+** CPE (cytopathic/cytotoxic effect) present **0** CPE (cytopathic/cytotoxic effect) not detected **NT** Not tested **N/A** Not applicable | | | | | | |

**TABLE 6**

| Test Product: PharmaPOVI Lot # 1/2012, 2/2012, 3/2012 | | | | | | |
|---|---|---|---|---|---|---|
| Virus / Strain: BVDV/ NADL ATCC #VR-534 | | | | | | |
| Host Cell Line: BT Host Cell Line ATCC #CRL-1390 | | | | | | |
| Volume Plated per Well: 1.0 mL | | | | | | |
| Exposure: 24 hours | | | | | | |
| **Dilution (-Log₁₀)** | **Control Product** | | | **Test Product** | | |
| | **Rep 1** | **Rep 2** | **Rep 3** | **Lot# 1/2012** | **Lot# 2/2012** | **Lot# 3/2012** |
| -2 | ++++ | ++++ | ++++ | 0000 | 0000 | 0000 |
| -3 | ++++ | ++++ | ++++ | 0000 | 0000 | 0000 |
| -4 | 0+++ | ++++ | ++++ | 0000 | 0000 | 0000 |
| -5 | ++++ | ++++ | ++++ | 0000 | 0000 | 0000 |
| -6 | 0000 | +000 | 0000 | 0000 | 0000 | 0000 |
| TCID₅₀ (Log₁₀) | 5.25 | 5.75 | 5.50 | ·1.50 | ·1.50 | ·1.50 |
| Average TCID₅₀ (Log₁₀) | 5.50 | | | <1.50 | | |
| Log₁₀ Reduction | NA | | | **·4.00** | **·4.00** | **·4.00** |
| Average Log₁₀ Reduction | NA | | | **·4.00** | | |
| Average Percent Reduction | NA | | | **·99.99**% | | |

| | | | | | | |
|---|---|---|---|---|---|---|
| **+** CPE (cytopathic/cytotoxic effect) present **0** CPE (cytopathic/cytotoxic effect) not detected **NT** Not tested **N/A** Not applicable | | | | | | |

**TABLE 7**

| Control Product: PharmaPOVI Untreated | | | | |
|---|---|---|---|---|
| Virus / Strain: BVDV / NADL ATCC #VR-534 | | | | |
| Host Cell Line: BT Host Cell Line ATCC #CRL-1390 | | | | |
| Volume Plated per Well: 1.0 mL | | | | |
| Exposure: 2 hours | | | | |
| Control vs. Initial Population | | | | |
| **Dilution (-Log₁₀)** | **Initial Population** | **Control Product** | | |
| | | **Replicate 1** | **Replicate 2** | **Replicate 3** |
| -2 | ++++ | ++++ | ++++ | ++++ |
| -3 | ++++ | ++++ | ++++ | ++++ |
| -4 | ++++ | ++++ | ++++ | ++++ |
| -5 | +++0 | ++0+ | ++++ | ++++ |
| -6 | 0+++ | +0+0 | 000+ | +0+0 |
| TCID₅₀ (Log₁₀) | 6.00 | 5.75 | 5.75 | 6.00 |
| Average TCID₅₀ (Log₁₀) | NA | 5.83 | | |
| Log₁₀ Reduction | NA | **0.25** | **0.25** | **0.00** |
| Average Log₁₀ Reduction | NA | **0.17** | | |
| Average Percent Reduction | NA | **32.39%** | | |

| | | | | |
|---|---|---|---|---|
| **+** CPE (cytopathic/cytotoxic effect) present **0** CPE (cytopathic/cytotoxic effect) not detected **NT** Not tested **N/A** Not applicable | | | | |

**TABLE 8**

| Control Product: PharmaPOVI Untreated | | | | |
|---|---|---|---|---|
| Virus / Strain: BVDV/ NADL ATCC #VR-534 BSLI Lot # 110311BVDV | | | | |
| Host Cell Line: BT Host Cell Line ATCC #CRL-1390 | | | | |
| BSLI Lot #092811BT | | | | |
| Volume Plated per Well: 1.0 mL | | | | |
| Exposure: 24 hours | | | | |
| Control vs. Initial Population | | | | |
| **Dilution (-Log₁₀)** | **Initial Population** | **Control Product** | | |
| | | **Replicate 1** | **Replicate 2** | **Replicate 3** |
| -2 | ++++ | ++++ | ++++ | ++++ |
| -3 | ++++ | ++++ | ++++ | ++++ |
| -4 | ++++ | 0+++ | ++++ | ++++ |
| -5 | +++0 | ++++ | ++++ | ++++ |
| -6 | 0+++ | 0000 | +000 | 0000 |
| ACID₅₀ (Log₁₀) | 6.00 | 5.25 | 5.75 | 5.50 |
| Average TCID₅₀ (Log₁₀) | NA | 5.50 | | |
| Log₁₀ Reduction | NA | **0.75** | **0.25** | **0.50** |
| Average Log₁₀ Reduction | NA | **0.50** | | |
| Average Percent Reduction | NA | **68.38%** | | |

| | | | | |
|---|---|---|---|---|
| **+** CPE (cytopathic/cytotoxic effect) present **0** CPE (cytopathic/cytotoxic effect) not detected **NT** Not tested **N/A** Not applicable | | | | |

**TABLE 9**

| Test Product: PharmaPOVI Lot # 1/2012, 2/2012, 3/2012 | | | | |
|---|---|---|---|---|
| Virus / Strain: BVDV/ NADL ATCC #VR-534 | | | | |
| Host Cell Line: BT Host Cell Line ATCC #CRL-1390 | | | | |
| Volume Plated per Well: 1.0 mL | | | | |
| Exposure: 2 hours | | | | |
| Test Product vs. Initial Population | | | | |
| **Dilution (-Log₁₀)** | **IP** | **Test Product** | | |
| | | **Lot# 1/2012** | **Lot# 2/2012** | **Lot# 3/2012** |
| -2 | ++++ | 0000 | 0000 | 0000 |
| -3 | ++++ | 0000 | 0000 | 0000 |
| -4 | ++++ | 0000 | 0000 | 0000 |
| -5 | +++0 | 0000 | 0000 | 0000 |
| -6 | 0+++ | 0000 | 0000 | 0000 |
| TCID50 | 6.00 | ·1.50 | ·1.50 | ·1.50 |
| Average TCID₅₀ | NA | | ·1.50 | |
| Log₁₀ Reduction | NA | ·4.50 | ·4.50 | ·4.50 |
| Average Log₁₀ Reduction | NA | ·4.50 | | |
| Average Percent Reduction | NA | >99.99% | | |

| | | | | |
|---|---|---|---|---|
| **+** CPE (cytopathic/cytotoxic effect) present **0** CPE (cytopathic/cytotoxic effect) not detected **NT** Not tested **N/A** Not applicable | | | | |

**TABLE 10**

| Test Product: PharmaPOVI Lot # 1/2012, 2/2012, 3/2012 | | | | |
|---|---|---|---|---|
| Virus / Strain: BVDV/ NADL ATCC #VR-534 | | | | |
| Host Cell Line: BT | | | | |
| Host Cell Line: ATCC #CRL-1390 | | | | |
| Volume Plated per Well: 1.0 mL | | | | |
| Exposure: 24 hours | | | | |
| Test Product vs. Initial Population | | | | |
| **Dilution (-Log₁₀)** | **IP** | **Test Product** | | |
| | | **Lot# 1/2012** | **Lot# 2/2012** | **Lot# 3/2012** |
| -2 | ++++ | 0000 | 0000 | 0000 |
| -3 | ++++ | 0000 | 0000 | 0000 |
| -4 | ++++ | 0000 | 0000 | 0000 |
| -5 | +++0 | 0000 | 0000 | 0000 |
| -6 | 0+++ | 0000 | 0000 | 0000 |
| TCID50 | 6.00 | ·1.50 | ·1.50 | ·1.50 |
| Average TCID₅₀ | NA | 1.50 | | |
| Log₁₀ Reduction | NA | **·4.50** | **·4.50** | **·4.50** |
| Average Log₁₀ Reduction | NA | **·4.50** | | |
| Average Percent Reduction | NA | **>99.99%** | | |

| | | | | |
|---|---|---|---|---|
| **+** CPE (cytopathic/cytotoxic effect) present **0** CPE (cytopathic/cytotoxic effect) not detected **NT** Not tested **N/A** Not applicable | | | | |

**TABLE 11**

| Test Product : PharmaPOVI Lot # 1/2012, 2/2012, 3/2012 | | | | | | |
|---|---|---|---|---|---|---|
| Control Product : PharmaPOVI Untreated | | | | | | |
| Virus / Strain : HIV-1/Mn ZeptoMetrix #: 0810027CF | | | | | | |
| Cell Line : C8166 | | | | | | |
| Host Cell Line ECACC # : 88051601 | | | | | | |
| Volume Plated per Well (mL): 1.0mL | | | | | | |
| Exposure: 2 hours | | | | | | |
| **Dilution (-Log₁₀)** | **Control Product** | | | **Test Product** | | |
| | **Rep 1** | **Rep 2** | **Rep 3** | **Lot# 1/2012** | **Lot# 2/2012** | **Lot# 3/2012** |
| -2 | ++++ | ++++ | ++++ | 0000 | 0000 | 0000 |
| -3 | ++++ | ++++ | ++++ | 0000 | 0000 | 0000 |
| -4 | ++++ | ++++ | ++++ | 0000 | 0000 | 0000 |
| -5 | +0++ | ++++ | 0+++ | 0000 | 0000 | 0000 |
| -6 | +++0 | ++0+ | ++0+ | 0000 | 0000 | 0000 |
| TCID50 | 5.75 | 6.25 | 6.00 | •1.50 | •1.50 | •1.50 |
| Average TCID₅₀ | 6.00 | | | 1.50 | | |
| Log₁₀ Reduction | NA | | | •4.50 | •4.50 | •4.50 |
| Average Log₁₀ Reduction | NA | | | •4.50 | | |
| Average Percent Reduction | NA | | | >99.99% | | |

| | | | | | | |
|---|---|---|---|---|---|---|
| **+** CPE (cytopathic/cytotoxic effect) present **0** CPE (cytopathic/cytotoxic effect) not detected **NT** Not tested **N/A** Not applicable | | | | | | |

**TABLE 12**

| Test Product : PharmaPOVI Lot # 1/2012, 2/2012, 3/2012 | | | | | | |
|---|---|---|---|---|---|---|
| Control Product : PharmaPOVI Untreated | | | | | | |
| Virus / Strain : HIV-1/Mn ZeptoMetrix #: 0810027CF | | | | | | |
| Cell Line : C8166 | | | | | | |
| Host Cell Line ECACC # : 88051601 | | | | | | |
| Volume Plated per Well (mL) : 1.0mL | | | | | | |
| Exposure : 24 hours | | | | | | |
| **Dilution (-Log₁₀)** | **Control Product** | | | **Test Product** | | |
| | **Rep 1** | **Rep 2** | **Rep 3** | **Lot# 1/2012** | **Lot# 2/2012** | **Lot# 3/2012** |
| -2 | ++++ | ++++ | ++++ | 0000 | 0000 | 0000 |
| -3 | ++++ | ++++ | ++++ | 0000 | 0000 | 0000 |
| -4 | ++++ | ++++ | ++++ | 0000 | 0000 | 0000 |
| -5 | +0++ | ++++ | +++0 | 0000 | 0000 | 0000 |
| -6 | 0000 | ++00 | +0+0 | 0000 | 0000 | 0000 |
| TCID50 | 5.25 | 6.00 | 5.75 | •1.50 | •1.50 | •1.50 |
| Average TCID₅₀ | 5.67 | | | •1.50 | | |
| Log₁₀ Reduction | NA | | | **•4.17** | •**4.17** | •**4.17** |
| Average Log₁₀ Reduction | NA | | | •**4.17** | | |
| Average Percent Reduction | NA | | | **>99.99**% | | |

| | | | | | | |
|---|---|---|---|---|---|---|
| **+** CPE (cytopathic/cytotoxic effect) present **0** CPE (cytopathic/cytotoxic effect) not detected **NT** Not tested **N/A** Not applicable | | | | | | |

**TABLE 13**

| Control Product : PharmaPOVI Untreated | | | | |
|---|---|---|---|---|
| Virus / Strain : HIV-1/Mn ZeptoMetrix #: 0810027CF | | | | |
| Cell Line : C8166 | | | | |
| Host Cell Line ECACC # : 88051601 | | | | |
| Volume Plated per Well (mL) : 1.0mL | | | | |
| Exposure : 2 hours | | | | |
| Control Product vs. Initial Population | | | | |
| **Dilution (-Log₁₀)** | **Initial Population** | **Control Product** | | |
| | | **Replicate 1** | **Replicate 2** | **Replicate 3** |
| -2 | NT | ++++ | ++++ | ++++ |
| -3 | ++++ | ++++ | ++++ | ++++ |
| -4 | ++++ | ++++ | ++++ | ++++ |
| -5 | ++++ | +0++ | ++++ | 0+++ |
| -6 | ++++ | +++0 | ++0+ | ++0+ |
| -7 | 0+++ | NT | NT | NT |
| TCID50 | 7.25 | 5.75 | 6.25 | 6.00 |
| Average TCID₅₀ | NA | 6.00 | | |
| Log₁₀ Reduction | NA | **1.50** | **1.00** | **1.25** |
| Average Log₁₀ Reduction | NA | **1.25** | | |
| Average Percent Reduction | NA | **94.38%** | | |

| | | | | |
|---|---|---|---|---|
| **+** CPE (cytopathic/cytotoxic effect) present **0** CPE (cytopathic/cytotoxic effect) not detected **NT** Not tested **N/A** Not applicable | | | | |

**TABLE 14**

| Test Product : PharmaPOVI Lot # 1/2012, 2/2012, 3/2012 | | | | |
|---|---|---|---|---|
| Virus / Strain : HIV-1/Mn ZeptoMetrix #: 0810027CF | | | | |
| Cell Line : C8166 | | | | |
| Host Cell Line ECACC # : 88051601 | | | | |
| Volume Plated per Well (mL) : 1.0mL | | | | |
| Exposure : 24 hours | | | | |
| Control Product vs. Initial Population | | | | |
| **Dilution (-Log₁₀**) | **IP** | Control Product | | |
| | | **Rep** 1 | **Rep 2** | **Rep 3** |
| -2 | NT | ++++ | ++++ | ++++ |
| -3 | ++++ | ++++ | ++++ | ++++ |
| -4 | ++++ | ++++ | ++++ | ++++ |
| -5 | ++++ | +0++ | ++++ | +++0 |
| -6 | ++++ | 0000 | ++00 | +0+0 |
| -7 | 0+++ | NT | NT | NT |
| TCID50 | 7.25 | 5.25 | 6.00 | 5.75 |
| Average TCID₅₀ | NA | 5.67 | | |
| Log₁₀ Reduction | NA | **2.00** | **1.25** | **1.5** |
| Average Log₁₀ Reduction | NA | **1.58** | | |
| Average Percent Reduction | NA | **>97.37**% | | |

| | | | | |
|---|---|---|---|---|
| **+** CPE (cytopathic/cytotoxic effect) present **0** CPE (cytopathic/cytotoxic effect) not detected **NT** Not tested **N/A** Not applicable | | | | |

**TABLE 15**

| Test Product : PharmaPOVI Lot # 1/2012, 2/2012, 3/2012 | | | | |
|---|---|---|---|---|
| Virus / Strain : HIV-1/Mn ZeptoMetrix #: 0810027CF ATCC or BSLI Lot #:111011HIV-1 | | | | |
| Cell Line : C8166 | | | | |
| Host Cell Line ECACC # : 88051601 ATCC or BSLI Lot # : 100803C8166 | | | | |
| Volume Plated per Well (mL) : 1.0mL | | | | |
| Exposure : 2 hours | | | | |
| Test Product vs. Initial Population | | | | |
| **Dilution (-Log₁₀)** | **Initial Population** | **Test Product** | | |
| | | **Lot# 1/2012** | **Lot# 2/2012** | **Lot# 3/2012** |
| -2 | NT | 0000 | 0000 | 0000 |
| -3 | ++++ | 0000 | 0000 | 0000 |
| -4 | ++++ | 0000 | 0000 | 0000 |
| -5 | ++++ | 0000 | 0000 | 0000 |
| -6 | ++++ | 0000 | 0000 | 0000 |
| -7 | 0+++ | NT | NT | NT |
| TCID50 | 7.25 | •1.50 | •1.50 | •1.50 |
| Average TCID₅₀ | NA | 1.50 | | |
| Log₁₀ Reduction | NA | •**5.75** | •**5.75** | •**5.75** |
| Average Log₁₀ Reduction | NA | •**5.75** | | |
| Average Percent Reduction | NA | **>99.99%** | | |

| | | | | |
|---|---|---|---|---|
| **+** CPE (cytopathic/cytotoxic effect) present **0** CPE (cytopathic/cytotoxic effect) not detected **NT** Not tested **N/A** Not applicable | | | | |

**TABLE 16**

| Test Product : PharmaPOVI Lot # 1/2012, 2/2012, 3/2012 | | | | |
|---|---|---|---|---|
| Virus/Strain : HIV-1/Mn ZeptoMetrix #: 0810027CF ATCC or BSLI Lot #:111011HIV-1 | | | | |
| Cell Line : C8166 | | | | |
| Host Cell Line ECACC # : 88051601 ATCC or BSLI Lot # : 100803C8166 | | | | |
| Volume Plated per Well (mL) : 1.0mL | | | | |
| Exposure : 24 hours | | | | |
| Test Product vs. Initial Population | | | | |
| **Dilution (-Log₁₀)** | **Initial Population** | **Test Product** | | |
| | | **Lot# 1/2012** | **Lot# 2/2012** | **Lot# 3/2012** |
| -2 | NT | 0000 | 0000 | 0000 |
| -3 | ++++ | 0000 | 0000 | 0000 |
| -4 | ++++ | 0000 | 0000 | 0000 |
| -5 | ++++ | 0000 | 0000 | 0000 |
| | | | | |
| -6 | ++++ | 0000 | 0000 | 0000 |
| -7 | 0+++ | NT | NT | NT |
| TCID50 | 7.25 | •1.50 | •1.50 | •1.50 |
| Average TCID₅₀ | NA | •1.50 | | |
| Log₁₀ Reduction | NA | •5.75 | •5.75 | •5.75 |
| Average Log₁₀ Reduction | NA | •5.75 | | |
| Average Percent Reduction | NA | >99.99% | | |

| | | | | |
|---|---|---|---|---|
| **+** CPE (cytopathic/cytotoxic effect) present **0** CPE (cytopathic/cytotoxic effect) not detected **NT** Not tested **N/A** Not applicable | | | | |

**TABLE 17**

| Test Product : PharmaPOVI Lot # 1/2012, 2/2012, 3/2012 | | | | | | |
|---|---|---|---|---|---|---|
| Virus / Strain : BVDV/ NADL ATCC #VR-534 | | | | | | |
| Host Cell Line : BT Host Cell Line ATCC #CRL-1390 | | | | | | |
| Volume Plated per Well : 1.0 mL | | | | | | |
| Neutralization and Cvtotoxicitv Control | | | | | | |
| **Dilution (-Log₁₀)** | **Neutralization Control** | | | **Cytotoxicity Control** | | |
| | **Lot# 1/2012** | **Lot# 2/2012** | **Lot# 3/2012** | **Lot# 1/2012** | **Lot# 2/2012** | **Lot# 3/2012** |
| -2 | ++++ | ++++ | ++++ | 0000 | 0000 | 0000 |
| -3 | ++++ | ++++ | ++++ | 0000 | 0000 | 0000 |
| -4 | ++++ | ++++ | ++++ | 0000 | 0000 | 0000 |
| -5 | ++++ | ++++ | ++++ | NT | NT | NT |
| -6 | +++0 | ++++ | ++00 | NT | NT | NT |
| -7 | +0+0 | +00+ | ++0+ | NT | NT | NT |
| TCID50 | 6.75 | 7.00 | 6.75 | •1.50 | •1.50 | •1.50 |
| Average TCID₅₀ | 6.83 | | | •1.50 | | |

| | | | | | | |
|---|---|---|---|---|---|---|
| **+** CPE (cytopathic/cytotoxic effect) present **0** CPE (cytopathic/cytotoxic effect) not detected **NT** Not tested **N/A** Not applicable | | | | | | |

**TABLE 18**

| Test Product : PharmaPOVI Lot # 1/2012, 2/2012, 3/2012 | | | | | | |
|---|---|---|---|---|---|---|
| Virus / Strain : HIV-1/Mn ZeptoMetrix #:0810027CF | | | | | | |
| Cell Line : C8166 Host Cell Line ECACC #:88051601 | | | | | | |
| Volume Plated per Well (mL) : 1.0mL | | | | | | |
| Neutralization and Cvtotoxicitv Control | | | | | | |
| **Dilution (-Log₁₀)** | **Neutralization Control** | | | **Cytotoxicity Control** | | |
| | **Lot# 1/2012** | **Lot# 2/2012** | **Lot# 3/2012** | **Lot# 1/2012** | **Lot# 2/2012** | **Lot# 3/2012** |
| -2 | ++++ | ++++ | ++++ | 0000 | 0000 | 0000 |
| -3 | ++++ | ++++ | ++++ | 0000 | 0000 | 0000 |
| -4 | ++++ | ++++ | ++++ | 0000 | 0000 | 0000 |
| -5 | ++++ | ++++ | ++++ | NT | NT | NT |
| -6 | +++0 | ++++ | ++00 | NT | NT | NT |
| -7 | +0+0 | +00+ | ++0+ | NT | NT | NT |
| TCID50 | 6.75 | 7.00 | 6.75 | •1.50 | •1.50 | •1.50 |
| Average TCID₅₀ | 6.83 | | | •1.50 | | |

| | | | | | | |
|---|---|---|---|---|---|---|
| **+** CPE (cytopathic/cytotoxic effect) present **0** CPE (cytopathic/cytotoxic effect) not detected **NT** Not tested **N/A** Not applicable | | | | | | |

### Conclusion

The wound dressing according to the present invention is substantially 100 % effective against the tested vira.

The study confirms that the inventive wound dressing laminate, which does not adhere to the wound dressing bed, can release the antimicrobial agent povidone-iodine despite the layers of the laminate are mechanically laminated to each other. The study confirms that the mechanical laminating procedures have no negative impact on the antiviral activity of povidone-iodine.

The wound dressings according to the present invention prevent infection of a wound, do not induce maceration, and absorb exudates at controlled level so as to continuously cleanse the wound bed without drying the wound. Manual cleansing during dressing changes, if any at all, is usually not necessary and significant nursing time can be saved.

## Claims

1. A wound dressing laminate (1') comprising a layer (3) impregnated with an antimicrobial agent (4), inserted between an apertured wound-contacting layer (2) and an absorbent non-woven layer (5;6), **characterised in that** the antimicrobial agent (4) is povidone-iodine, and
the layer (3) impregnated with povidone-iodine (4) and the absorbent non-woven layer (5) are a coherent double-ply structure laminated together by needle punching.

2. A wound dressing laminate (1') according to claim 1, **characterised in that** the apertured wound-contacting layer (2) is a non-adherent polyolefin film, preferably the polyolefin is polyethylene or polypropylene, or a non-adherent polyurethane film or polyester film, optionally the apertured wound-contacting layer (2) is a film layer selected from a perforated film, a net, a scrim or a mesh.

3. A wound dressing laminate (1') according to any of the preceding claims 1 or 2, **characterised in that** the absorbent non-woven layer (5;6) comprises one or more of the absorbent components selected from the group comprising viscose fibers, polyolefin fibers, superabsorbent fibers and gel-forming fibers or combinations thereof,
- optionally the polyolefin fibers of the absorbent non-woven layer (5;6) is selected from the group comprising polyester fibers, polypropylene fibers and bico fibers, or combinations thereof,
- optionally the absorbent components include one or more of carboxymethyl cellulose fibers and calcium alginate fibers, or combinations thereof,
- optionally the absorbent non-woven layer (5;6) is coated with powdered superabsorbent polymer (33),
- optionally the superabsorbent fibers are cross-linked acrylate copolymer fibers,
- optionally the absorbent non-woven layer (5;6) is composed substantially exclusively of viscose fibers.

4. A wound dressing laminate (1') according to claim 3, **characterised in that** the ratio of viscose fibers to polyolefin fibers in the absorbent non-woven layer (5; 6) is 100:0 wt% based on the total weight of viscose fibers and polyolefin fibers, alternatively 75:25 wt% based on the total weight of viscose fibers and polyolefin fibers, alternatively 50:50 wt% based on the total weight of viscose fibers, alternatively 25:75 wt% based on the total weight of viscose fibers and polyolefin fibers and polyolefin fibers, and most preferred 75:25 wt% based on the total weight of viscose fibers and polyolefin fibers.

5. A wound dressing laminate (1') according to claim 4, **characterised in that** the superabsorbent fibers and/or gel-forming fibres of the absorbent non-woven layer (5;6) is provided in an amount that provides the wound dressing laminate (1') with an absorption capacity of at least 20, more preferred at least 25, even more preferred at least about 30, and most preferred up to 40 g fluid per g wound dressing laminate.

6. A wound dressing laminate (1') according to any of the preceding claims 1 - 5, **characterised in that** the layer (3) impregnated with antimicrobial agent (4) is a knitted fabric, a net or mesh,
- optionally the layer impregnated (3) with antimicrobial agent (4) is a viscose layer, optionally the layer impregnated (3) with antimicrobial agent (4) is impregnated with an ointment, wherein the antimicrobial agent (4) represents 1 - 10 wt% of the total weight of the ointment,
- preferably the antimicrobial agent (4) of the impregnated layer (3) is povidone-iodine in form of a polyglycol/povidone-iodine ointment, wherein the povidone-iodine represent 1 - 10 wt% povidone-iodine of the total weight of the ointment.

7. A wound dressing laminate (1') according to any of the preceding claims 1 - 6, **characterised in that** the secondary absorbent layer (6) is laminated to the absorbent non-woven layer (5), preferably the secondary absorbent layer (6) has same or different composition as the absorbent non-woven layer (5).

8. A method of manufacturing a wound dressing laminate (1') comprising a layer (3) impregnated with antimicrobial agent (4),
**characterised in that** the method comprising the steps of
- providing the layer (3) impregnated with an antimicrobial agent (4) in form of povidone-iodine,
- providing an apertured wound-contacting layer (2) adjacent one side of the layer (3) impregnated with the antimicrobial agent (4),
- providing an absorbent non-woven layer (5) adjacent the opposite side of the layer (3) impregnated with the antimicrobial agent (4),
- the layer (3) impregnated with the antimicrobial agent (4) and the absorbent non-woven layer (5) are laminated to a coherent double-ply structure using needle punching, and
- laminating the layers (2;3;5) together.

9. A method according to claim 8, **characterised in that** the apertured wound-contacting layer (2) is laminated to the layer (3) impregnated with antimicrobial agent (4) at the side free of absorbent non-woven layer (5) to obtain a coherent triple-ply structure using a second mechanical bonding step.

10. A method according to claim 9, **characterised in that** the second mechanical bonding step is calendaring or needle punching.

11. A method according to any of the preceding claims 8 - 10, **characterised in that** the layer (3) impregnated with antimicrobial agent (4) is manufactured by passing a layer (10) of non-impregnated fabric through a bath (13) with a mixture of antimicrobial agent (4) and allowing the antimicrobial agent to deposit on the impregnated fabric (10,3,4), preferably the antimicrobial agent (4) is povidone-iodine and the layer (3) impregnated with povidone-iodine is manufactured by passing a layer (10) of non-impregnated fabric through a heated bath 13 with a mixture of polyethylen glycol and povidone-iodine and allowing the heated impregnated fabric (3) to cool.

12. A method according to any of the preceding claims 8 - 11, **characterised in that** an absorbent non-woven layer (5; 6) is manufactured by
a) providing a blend (M1;M2) of one or more fiber components selected from the group comprising viscose fibers, polyolefin fibers and/or superabsorbent fibers,
b) carding the fiber blend (M1;M2) to obtain a web (22) of mixed fiber components,
c) passing said web (22) through a cross lapper (21) after carding the fiber blend ((M1;M2) in step b),
d) needle punching the web from at least one side, and
e) calendering the needle punched web to obtain the absorbent non-woven layer laminate (5;6).

13. A method according to any of the preceding claims 8 - 12, **characterised in that** the method further comprises the step of
f) coating the absorbent non-woven layer (5;6) with powders (33) coated with superabsorbent polymer.

14. A method according to any of the preceding claims 8 - 13, **characterised in that** the method further comprises the step of laminating the absorbent non-woven layer (5) side of the wound dressing laminate (1') to a layer of secondary absorbent dressing (6), optionally the secondary layer (6) of absorbent dressing has a higher absorptive capacity than the absorbent non-woven layer (5), optionally the secondary layer of absorbent dressing (6) is a foam layer, preferably of polyurethane.

15. A wound dressing (1) **characterised in that** it includes a pad of the wound dressing laminate (1') according to any of the preceding claims 1 - 7, optionally the periphery of the wound dressing is sealed using a mechanical sealing step, preferably the mechanical sealing step is ultrasonic welding or heat sealing.

16. A wound dressing (1) according to claim 15, **characterised in that** the wound dressing (1) is an island pad wound dressing.

## Patentansprüche

1. Wundverbandslaminat (1') mit einer Schicht (3), die mit einem antimikrobiellen Wirkstoff (4) imprägniert ist, die zwischen einer offenen Wundkontaktschicht (2) und einer absorbierenden Vliesschicht (5; 6) eingelegt ist, **dadurch gekennzeichnet, dass** der antimikrobielle Wirkstoff (4) Povidon-Jod (4) ist und die Schicht, die mit
Povidon-Jod (4) imprägniert ist, und die absorbierende Vliesschicht (5) eine kohärente doppellagige Struktur sind, die durch Vernadeln laminiert sind.

2. Wundverbandslaminat (1') nach Anspruch 1, **dadurch gekennzeichnet, dass** die offene Wundkontaktschicht (20) ein nicht-anhaftender Polyolefinfilm, wobei das Polyolefin bevorzugt Polyethylen oder Polypropylen oder ein nicht-anhaftender Polyurethanfilm oder Polyesterfilm ist, wobei die offene Wundkontaktschicht (2) optional eine Filmschicht ist, ausgewählt aus einem perforierten Film, einem Netz, einem Gitterstoff oder einem Netz.

3. Wundverbandslaminat (1') nach einem der vorhergehenden Ansprüche 1 oder 2,
**dadurch gekennzeichnet, dass** die absorbierende Vliesschicht (5; 6) eine oder mehrere absorbierende Komponenten aufweist, ausgewählt aus der Gruppe bestehend aus Viskosefasern, Polyolefinfasern, superabsorbierenden Fasern und Gel-bildenden Fasern oder Kombinationen dieser, wobei
- die Polyolefinfasern der absorbierenden Vliesschicht (5; 6) optional ausgewählt sind aus der Gruppe bestehend aus Polyesterfasern, Polypropylenfasern und Bicofasern oder Kombinationen dieser,
- die absorbierenden Komponenten optional eine oder mehrere von Carboxymethylcellulosefasern und Calciumalginatfasern oder Kombinationen dieser enthalten,
- die absorbierende Vliesschicht (5; 6) optional mit pulverförmigem superabsorbierendem Polymer (33) beschichtet ist,
- die superabsorbierenden Fasern optional quervernetzte Acrylpolymerfasern sind,
- die absorbierende Vliesschicht (5; 6) optional im Wesentlichen ausschließlich aus Viskosefasern aufgebaut ist.

4. Wundverbandslaminat (1') nach Anspruch 3, **dadurch gekennzeichnet, dass** das Verhältnis von Viskosefasern zu Polyolefinfasern in der absorbierenden Vliesschicht (5; 6) 100:0 Gewichtsprozent ist, basierend auf dem Gesamtgewicht der Viskosefasern und der Polyolefinfasern, alternativ 75:25 Gewichtsprozent, basierend auf dem Gesamtgewicht der Viskosefasern und der Polyolefinfasern, alternativ 50:50 Gewichtsprozent, basierend auf dem Gesamtgewicht der Viskosefasern, alternativ 25:75 Gewichtsprozent, basierend auf dem Gesamtgewicht der Viskosefasern und der Polyolefinfasern und Polyolefinfasern und höchst bevorzugt 75:25 Gewichtsprozent, basierend auf dem Gesamtgewicht der Viskosefasern und Polyolefinfasern.

5. Wundverbandsschicht (1') nach Anspruch 4, **dadurch gekennzeichnet, dass** die superabsorbierenden Fasern und/oder gelbildenden Fasern der absorbierenden Vliesschicht (5; 6) in einer Menge bereitgestellt sind, die das Wundverbandslaminat (1') mit einer Absorptionskapazität von wenigstens 20, bevorzugt von wenigstens 25, stärker bevorzugt von wenigstens 30 und höchst bevorzugt von bis zu 40 g Fluid pro g Wundverbandslaminat ausstattet.

6. Wundverbandslaminat (1') nach einem der vorhergehenden Ansprüche 1-5, **dadurch gekennzeichnet, dass** die Schicht (3), die mit dem antimikrobiellen Wirkstoff (4) imprägniert ist, ein gestricktes Gewebe, ein Netz oder ein Geflecht ist, wobei
- die Schicht (3), die mit dem antimikrobiellen Wirkstoff (4) imprägniert ist, optional eine Viskoseschicht ist, wobei die Schicht (3), die mit dem antimikrobiellen Wirkstoff (4) imprägniert ist, optional mit einer Salbe imprägniert ist, wobei der antimikrobielle Wirkstoff (4) 1 - 10 Gewichtsprozent des Gesamtgewichts der Salbe darstellt,
- der antimikrobielle Wirkstoff (4) der imprägnierten Schicht (3) bevorzugt Povidon-Jod in Form einer Polyglykol/Povidon-Jod-Salbe ist, wobei das Povidon-Jod 1 - 10 Gewichtsprozent Povidon-Jod des Gesamtgewichts der Salbe darstellt.

7. Wundverbandslaminat (1') nach einem der vorhergehenden Ansprüche 1-6, **dadurch gekennzeichnet, dass** die zweite absorbierende Schicht (6) auf die absorbierende Vliesschicht (5) laminiert ist, wobei die zweite absorbierende Schicht (6) bevorzugt dieselbe oder eine andere Zusammensetzung als die absorbierende Vliesschicht (5) hat.

8. Verfahren zum Herstellen eines Wundverbandslaminats (1') mit einer Schicht (3), die mit einem antimikrobiellen Wirkstoff (4) imprägniert ist, **dadurch gekennzeichnet, dass** das Verfahren die Schritte aufweist:
- Bereitstellen der Schicht (3), die mit einem antimikrobiellen Wirkstoff (4) in Form von Povidon-Jod imprägniert ist,
- Bereitstellen einer offenen Wundkontaktschicht (2), die einer Seite der Schicht (3), die mit dem antimikrobiellen Wirkstoff (4) imprägniert ist, benachbart ist.
- Bereitstellen einer absorbierenden Vliesschicht (5), die der gegenüber liegenden Seite der Schicht (3), die mit dem antimikrobiellen Wirkstoff (4) imprägniert ist, benachbart ist,
- wobei die Schicht (3), die mit dem antimikrobiellen Wirkstoff (4) imprägniert ist und die absorbierende Vliesschicht (5) zu einer kohärenten zweilagigen Schicht durch Vernadeln laminiert sind, und
- Laminieren der Schichten (2; 3; 5).

9. Verfahren nach Anspruch 8, **dadurch gekennzeichnet, dass** die offenen Wundkontaktschicht (2) auf die Schicht (3), die mit dem antimikrobiellen Wirkstoff (4) imprägniert ist, auf der Seite, die frei von der absorbierenden Schicht (5) ist, laminiert wird, um eine kohärente dreilagige Struktur durch Verwenden eines zweiten mechanischen Verbindungsschritts zu erhalten.

10. Verfahren nach Anspruch 9, **dadurch gekennzeichnet, dass** der zweite mechanische Verbindungsschritt Kalandrieren oder Vernadeln ist.

11. Verfahren nach einem der vorgehenden Ansprüche 8 - 10, **dadurch gekennzeichnet, dass** die Schicht (3), die mit dem antimikrobiellen Wirkstoff (4) imprägniert ist, durch Passieren einer Schicht (10) eines nichtimprägnierten Gewebes durch ein Bad (13) mit einer Mixtur eines antimikrobiellen Wirkstoffs (4) und Ablagern lassen des antimikrobiellen Wirkstoffs auf dem imprägnierten Gewebe (10, 3, 4) hergestellt wird, wobei der antimikrobielle Wirkstoff (4) bevorzugt Povidon-Jod ist und die Schicht (3), die mit Povidon-Jod imprägniert wird, hergestellt wird durch Passieren einer Schicht (10) eines nichtimprägnierten Gewebes durch ein erwärmtes Bad (13) mit einer Mischung aus Polyethylenglykol und Povidon-Jod und Abkühlen lassen des erwärmten imprägnierten Gewebes (3).

12. Verfahren nach einem der vorhergehenden Ansprüche 8 - 11, **dadurch gekennzeichnet, dass** eine absorbierende Vliesschicht (5; 6) hergestellt wird durch
a) Bereitstellen einer Mischung (M1; M2) einer oder mehrerer Faserkomponenten, die ausgewählt sind aus der Gruppe, bestehend aus Viskosefasern, Polyolefinfasern und/oder superabsorbierenden Fasern,
b) Kardieren der Fasermischung (M1; M2), um ein Gewebe (22) der gemischten Faserkomponenten zu erhalten,
c) Passieren des Gewebes (22) durch einen Querleger (21) nach dem Karieren der Fasermischung (M1; M2) in Schritt b),
d) Vernadeln des Gewebes von wenigstens einer Seite, und
e) Kalandrieren des vernadelten Gewebes, um ein absorbierendes Vliesschichtlaminat (5; 6) zu erhalten.

13. Verfahren nach einem der vorhergehenden Ansprüche 8 - 12, **dadurch gekennzeichnet, dass** das Verfahren weiter den Schritt aufweist:
f) Beschichten der absorbierenden Vliesschicht (5; 6) mit Pulvern (33), die mit superabsorbierendem Polymer beschichtet sind.

14. Verfahren nach einem der vorhergehenden Ansprüche 8 - 13, **dadurch gekennzeichnet, dass** das Verfahren weiter die Schutte des Laminierens der Seite der absorbierenden Vliesschicht (5) des Wundverbandslaminats (1') auf eine Schicht eines zweiten absorbierenden Verbands (6) aufweist, wobei die zweite Schicht (6) des Wundverbands optional eine höhere Absorptionskapazität als die absorbierende Vliesschicht (5) hat, wobei die zweite Schicht des absorbierenden Verbands (6) optional eine geschäumte Schicht bevorzugt aus Polyurethan ist.

15. Wundverband (1), **dadurch gekennzeichnet, dass** dieser ein Kissen des Wundverbandlaminats (1') gemäß einem der vorhergehenden Ansprüche 1 - 7 aufweist, wobei die Peripherie des Wundverbands optional durch Verwenden eines mechanischen Versiegelungsverfahrens versiegelt ist, wobei der mechanische Versiegelungsschritt bevorzugt Ultraschallschweißen oder Wärmeversiegeln ist.

16. Wundverband (1) nach Anspruch 15, **dadurch gekennzeichnet, dass** der Wundverband (1) ein Inselkissenwundverband ist.

## Revendications

1. Stratifié (1') sous la forme d'un pansement pour blessures comprenant une couche (3) imprégnée avec un agent antimicrobien (4), insérée entre une couche munie d'orifices (2) qui entre en contact avec la peau et une couche absorbante (5 ; 6) sous la forme d'un non tissé, **caractérisé en ce que** l'agent antimicrobien (4) est de la povidone iodée (4), et la couche (3) imprégnée avec de la povidone iodée (4) ainsi que la couche absorbante (5) sous la forme d'un non tissé représentent une structure cohérente bicouche cohérente contrecollées l'une à l'autre par aiguilletage.

2. Stratifié (1') sous la forme d'un pansement pour blessures selon la revendication 1, **caractérisé en ce que** la couche munie d'orifices (2) qui entre en contact avec la peau représente un film de polyoléfine non adhérent, la polyoléfine représentant de préférence du polyéthylène ou du polypropylène, ou un film de polyester ou un film de polyuréthane non adhérent, la couche munie d'orifices (2) qui entre en contact avec la peau représentant de manière facultative une couche pelliculaire choisie parmi un film perforé, un filet, une gaze ou une maille.

3. Stratifié (1') sous la forme d'un pansement pour blessures selon l'une quelconque des revendications 1 ou 2, **caractérisé en ce que** la couche absorbante (5 ; 6) sous la forme d'un non tissé comprend un ou plusieurs des composants absorbants choisis parmi le groupe comprenant des fibres de viscose, des fibres de polyoléfine, des fibres superabsorbantes et des fibres gélifiantes, ou leurs combinaisons ;
- les fibres de polyoléfine de la couche absorbante (5 ; 6) sous la forme d'un non tissé étant de manière facultative choisies parmi le groupe comprenant des fibres de polyester, des fibres de polypropylène et des fibres à deux composants, ou leurs combinaisons ;
- les composants absorbants englobant de manière facultative une ou plusieurs fibres choisies parmi le groupe comprenant des fibres de carboxyméthylcellulose et des fibres d'alginate de calcium, ou leurs combinaisons ;
- la couche absorbante (5 ; 6) sous la forme d'un non tissé étant de manière facultative enduite d'un polymère pulvérulent superabsorbant (33) ;
- les fibres superabsorbantes représentant de manière facultative des fibres réticulées de copolymères à base d'acrylate ;
- la couche absorbante (5 ; 6) sous la forme d'un non tissé étant de manière facultative composée de manière essentiellement exclusive de fibres de viscose.

4. Stratifié (1') sous la forme d'un pansement pour blessures selon la revendication 3, **caractérisé en ce que** le rapport des fibres de viscose aux fibres de polyoléfine dans la couche absorbante (5 ; 6) sous la forme d'un non tissé s'élève à 100:0 % en poids, basés sur le poids total des fibres de viscose et des fibres de polyoléfine, en variante à 75:25 % en poids, basés sur le poids total des fibres de viscose et des fibres de polyoléfine, en variante à 50:50 % en poids, basés sur le poids total des fibres de viscose et des fibres de polyoléfine, en variante à 25:75 % en poids, basés sur le poids total des fibres de viscose et des fibres de polyoléfine, et de manière de loin préférée à 75:25 % en poids, basés sur le poids total des fibres de viscose et des fibres de polyoléfine.

5. Stratifié (1') sous la forme d'un pansement pour blessures selon la revendication 4, **caractérisé en ce que** les fibres superabsorbantes et/ou les fibres gélifiantes de la couche absorbante (5 ; 6) sous la forme d'un non tissé sont fournies en une quantité qui confère au stratifié (1') sous la forme d'un pansement pour blessures une capacité d'absorption d'au moins 20, de manière plus préférée d'au moins 25, de manière encore plus préférée d'au moins environ 30 et de manière de loin préférée jusqu'à 40 g de fluide par g de stratifié sous la forme d'un pansement pour blessures.

6. Stratifié (1') sous la forme d'un pansement pour blessures selon l'une quelconque des revendications précédentes 1 à 5, **caractérisé en ce que** la couche (3) imprégnée avec un agent antimicrobien (4) représente un tissu tricoté, un filet ou une maille ;
- la couche (3) imprégnée avec un agent antimicrobien (4) représentant de manière facultative une couche de viscose;
- la couche (3) imprégnée avec un agent antimicrobien (4) étant de manière facultative imprégnée avec un onguent, l'agent antimicrobien (4) représentant de 1 à 10 % en poids du poids total de l'onguent ;
- l'agent antimicrobien (4) de la couche imprégnée (3) représentant de préférence de la povidone iodée sous la forme d'un onguent de polyglycol/povidone iodée, la povidone iodée représentant de 1 à 10 % en poids de povidone iodée du poids total de l'onguent.

7. Stratifié (1') sous la forme d'un pansement pour blessures selon l'une quelconque des revendications précédentes 1 à 6, **caractérisé en ce que** la couche absorbante secondaire (6) est contrecollée à la couche absorbante (5) sous la forme d'un non tissé, la couche absorbante secondaire (6) possédant de préférence une composition identique à ou différente de celle de la couche absorbante (5) sous la forme d'un non tissé.

8. Procédé de confection d'un stratifié (1') sous la forme d'un pansement pour blessures comprenant une couche (3) imprégnée avec un agent antimicrobien (4), **caractérisé en ce que** le procédé comprend les étapes consistant à :
- procurer la couche (3) imprégnée avec un agent antimicrobien (4) sous la forme de povidone iodée ;
- procurer une couche munie d'orifices (2) qui entre en contact avec la peau en position adjacente à un côté de la couche (3) imprégnée avec l'agent antimicrobien (4) ;
- procurer une couche absorbante (5) sous la forme d'un non tissé en position adjacente au côté opposé de la couche (3) imprégnée avec l'agent antimicrobien (4) ;
- contrecoller la couche (3) imprégnée avec l'agent antimicrobien (4) et la couche absorbante (5) sous la forme d'un non tissé pour obtenir une structure bicouche cohérente en utilisant un aiguilletage ; et
- contrecoller les couches (2, 3, 5) les unes aux autres.

9. Procédé selon la revendication 8, **caractérisé en ce que** la couche munie d'orifices (2) qui entre en contact avec la peau est contrecollée à la couche (3) imprégnée avec un agent antimicrobien (4) du côté exempt de la couche absorbante (5) sous la forme d'un non tissé pour obtenir une structure cohérente triple couche en utilisant une deuxième étape de collage mécanique.

10. Procédé selon la revendication 9, **caractérisé en ce que** la deuxième étape de collage mécanique est un calandrage ou un aiguilletage.

11. Procédé selon l'une quelconque des revendications précédentes 8 à 10, **caractérisé en ce qu'**on fabrique la couche (3) imprégnée avec un agent antimicrobien (4) en faisant passer une couche (10) constituée d'un tissu non imprégné à travers un bain (13) comprenant un mélange d'agent antimicrobien (4) et en laissant l'agent antimicrobien se déposer sur le tissu imprégné (10, 3, 4), l'agent antimicrobien (4) représentant de préférence de la povidone iodée et la couche (3) imprégnée avec la povidone iodée étant fabriquée en faisant passer une couche (10) constituée d'un tissu non imprégné à travers un bain chauffé (13) comprenant un mélange de polyéthylèneglycol et de povidone iodée, et en laissant refroidir le tissu imprégné chauffé (3).

12. Procédé selon l'une quelconque des revendications 8 à 11, **caractérisé en ce qu'**on fabrique une couche absorbante (5 ; 6) sous la forme d'un non tissé :
a) en procurant un mélange (M1; M2) d'un ou de plusieurs composants fibreux choisis parmi le groupe comprenant des fibres de viscose, des fibres de polyoléfine et/ou des fibres superabsorbantes ;
b) en soumettant le mélange fibreux (M1; M2) à un cardage pour obtenir une bande (22) constituée par des composants fibreux mélangés ;
c) en faisant passer ladite bande (22) à travers une nappeuse transversale (21) après le cardage du mélange fibreux (M1; M2) à l'étape b) ;
d) en soumettant la bande à un aiguilletage à partir d'au moins un côté ; et
e) en soumettant la bande aiguilletée à un calandrage pour obtenir le stratifié de couche absorbante (5 ; 6) sous la forme d'un non tissé.

13. Procédé selon l'une quelconque des revendications 8 à 12, **caractérisé en ce que** le procédé comprend en outre l'étape consistant à :
f) recouvrir la couche absorbante (5 ; 6) sous la forme d'un non tissé avec des poudres (33) enduites d'un polymère superabsorbant.

14. Procédé selon l'une quelconque des revendications 8 à 13, **caractérisé en ce que** le procédé comprend en outre l'étape consistant à contrecoller le côté du stratifié (1') sous la forme d'un pansement pour blessures concerné par la couche absorbante (5) sous la forme d'un non tissé à une couche d'un pansement absorbant secondaire (6), la couche secondaire (6) du pansement absorbant possédant de manière facultative une capacité d'absorption supérieure à celle de la couche absorbante (5) sous la forme d'un non tissé, la couche secondaire du pansement absorbant (6) représentant de manière facultative une couche en mousse, de préférence de polyuréthane.

15. Pansement pour blessures (1) **caractérisé en ce qu'**il englobe un tampon constitué du stratifié (1') sous la forme d'un pansement pour blessures selon l'une quelconque des revendications précédentes 1 à 7, la périphérie du pansement pour blessures étant de manière facultative scellée en utilisant une étape de scellement mécanique, l'étape de scellement mécanique représentant de préférence une soudure aux ultrasons ou un thermosoudage.

16. Pansement pour blessures (1) selon la revendication 15, **caractérisé en ce que** le pansement pour blessures (1) est un pansement pour blessures comprenant un tampon sous la forme d'une île.
